# EUROPÄISCHE PATENTSCHRIFT

(11) **EP 1 549 281 B1**
(45) Veröffentlichungstag und Bekanntmachung des Hinweises auf die Patenterteilung: **17.01.2007**
(21) Anmeldenummer: 03773636.0
(22) Anmeldetag: 29.09.2003
(51) Int. Cl.: A61K 8/04, A61K 8/34, A61K 8/37, A61K 8/39, A61K 8/73, A61K 8/86, A61Q 5/00, A61Q 17/04, A61Q 19/00, A61Q 19/02

(54) **ALS AEROSOL VERSPRÜHBARE W/O-EMULSIONEN**
W/O EMULSIONS SPRAYED AS AN AEROSOL
EMULSIONS EAU-HUILE PULVERISABLES SOUS FORME D'AEROSOL

(30) Priorität: 01.10.2002 DE 10245727
(43) Veröffentlichungstag der Anmeldung: 06.07.2005
(73) Patentinhaber: Beiersdorf AG, 20245 Hamburg (DE)
(72) Erfinder: SCHREIBER, Jörg, 22087 Hamburg (DE); MAURER, Peter, 22880 Wedel (DE); MIERTSCH, Heike, 22529 Hamburg (DE)
(86) Internationale Anmeldenummer: PCT/EP2003/010811
(87) Internationale Veröffentlichungsnummer: WO 2004/030641

(56) Entgegenhaltungen:
- WO-A-98/15254
- FR-A- 2 320 729
- US-A1- 2002 082 327
- PATENT ABSTRACTS OF JAPAN vol. 2000, no. 06, 22. September 2000 (2000-09-22) & JP 2000 080017 A (KANEBO LTD), 21. März 2000 (2000-03-21)

## Beschreibung

Die vorliegende Erfindung betrifft als Aerosol versprühbare kosmetische und dermatologische W/O-Emulsionen, insbesondere hautpflegende als Aerosol versprühbare kosmetische und dermatologische W/O-Emulsionen.

Ein Aerosol ist ein disperses System, bei dem ein Feststoff oder eine Flüssigkeit in einem Gas äußerst fein verteilt vorliegt. Das Aerosol wird in der Regel erst bei der Anwendung mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils entweicht das Treibmittel-Zubereitungsgemisch durch eine feine Düse, das Treibmittel verdampft und hinterläßt das fein verteilte Sprühgut als Aerosol.

Wirkstoffe können in Aerosolformulierungen sowohl gelöst als auch in fester Form vorliegen; liegen sie in fester Form vor, müssen sie allerdings im Treibmittelsystem entsprechend suspendiert werden.

Als Aerosol versprühbare kosmetische und dermatologische Hautpflegezubereitungen auf Emulsionsbasis sind in der Regel O/W-Systeme, in denen hydrophile Wirkstoffe in der externen Wasserphase gelöst sind. Die Ölphase enthält häufig silikonhaltige Öle, welche zu einem angenehmen Hautgefühl nach dem Versprühen aus einem Aerosolbehältnis beitragen.

Allerdings haben Sprühgüter in Form von O/W-Formulierungen den Nachteil, dass sie auf eine beschränkte Rohstoffauswahl begrenzt sind, da nur bei Verwendung hydrophiler Treibgase - wie z. B. Kohlendioxid - wirklich Aerosole entstehen. Werden hingegen übliche lipophile Treibmittel wie beispielsweise Kohlenwasserstoffe eingesetzt, so wird beim Austreten aus der Düse im allgemeinen ein Schaum erzeugt, da diese Treibmittel sich in der Ölphase der Formulierung lösen und somit aus der inneren Phase der Emulsion verdampfen.

Ein weiterer Nachteil derartiger Formulierungen - bei denen die Wasserphase die äußere Phase darstellt - ist, dass diese die in der Regel als Behälter verwendeten Metalldosen korrodieren können, und zwar umso stärker je höher die Korrosivität eingesetzter Hilfs-, Zusatz- und/oder Wirkstoffe ist. Es ist daher üblich und in der Regel notwendig, die eingesetzten Dosen von innen zu lackieren.

Wasserhaltige Aerosol-Formulierungen auf Basis von W/O-Emulsionen - die die obengenannten Nachteile nicht zeigen würden - sind bisher im Markt nicht bekannt. Bei der Entwicklung derartiger Zubereitungen treten ein Reihe von besonderen Problemen auf, beispielsweise dass die Ölphase sich als äußere Phase zu erheblichen Anteilen im Treibmittel lösen kann. Ferner muß bei der Formulierung beachtet werden, dass das Treibgas, welches im allgemeinen erst beim Abfüllen der Zubereitung zugesetzt wird, einen Einfluß auf die physikalischen Eigenschaften des Produktes hat. So kann der Zusatz des Treibmittels z. B. eine Phasentrennung auslösen, so dass keine homogene Versprühung des Füllgutes möglich ist, oder die Viskosität des Produktes so verändern, dass es gar nicht erst sprühbar ist. Eine Verträglichkeitsprüfung des Originalproduktes mit dem Treibmittel ist daher nahezu unerläßlich.

Ferner können klassisch formulierte W/O-Emulsionen auch unkosmetisch bei der Anwendung sein.

Antitranspirant-Kombinationen auf Basis von silikonhaltigen "W/O" bzw. W/S-Emulsionen sind an sich bekannt. In der WO 9624326 wird eine Zubereitung mit 10 bis 50 % einer W/O-Emulsion und 50 bis 90 % eines Treibgases beschrieben, die ein Aluminiumsalz enthält.

Die WO 93200984 beschreibt ähnliche Aerosole auf Basis von silikonhaltigen Wasser-in-Öl Mikroemulsionen, die nach dem Verdampfen des Treibgases zu klaren Gelen auf der Haut führen. Allerdings wird hier die Solubilisierung von Wasser in einem kosmetischen Öl (Silikonöl, Esteröle, Ether, langkettige Alkohole, Triglyceride) in Gegenwart von silikonfreien Emulgatoren zu versprühbaren W/O-Emulsionen nicht beschrieben.

Der Stand der Technik kennt keine W/O-Emulsionen mit silikonölfreien Ölen oder silikonölfreien Emulgatoren, welche sich zu Aerosolen versprühen ließen.

In der EP1013268 wird beschrieben, wie Emulsionen und Liposomen in getrennten Behältnissen zu einem Aerosol versprüht werden können. Die getrennte Herstellung beider Systeme mit anschließender Vereinigung in einem Aerosolbehältnis ist allerdings zeitaufwendig und teuer.

In der US 4439343 werden Aerosolzubereitungen beschrieben, die Cocodiethanolamid als Emulgator enthalten. Nachteilig ist die für leave-on Produkte zu geringe Verträglichkeit von Zubereitungen, welche diesen Emulgator enthalten.

Die US 4695451 beschreibt Aerosol-Zubereitungen auf der Basis von W/O-Emulsionen. Als Emulsionsstabilisator wird eine Kombination von C3-C4 Estern von C12-C18 Alkansäuren (Seite 4, Zeile 19-21, Seite 4, Zeile 44-52, Seite 4, Zeile 59-60) (besonders bevorzugt Isopropylmyristat oder Isopropylpalmitat) mit einem zweiten, treibgaslöslichen Stabilisator (Seite 4, Zeile 61-68) (bevorzugt C8- Isoparaffin) verwendet. Ferner können auch Silikonöle als Stabilisatoren eingesetzt werden (Seite 4, Zeile 51-52), wobei Phenylmethyl Polysiloxane bzw. Dimethylsiloxane bevorzugt sind. Nachteil dieser Zubereitungen ist, dass sowohl Isopropylverbindungen als auch kurzkettige Isoparaffine häufig zu Hautunverträglichkeiten oder auch zu verstärkter Penetration von weiteren Inhaltstoffen (Parfümbestandteile, Konservierungsmittel) führen können. Cyclische Silikonöle in Gegenwart silikonfreier Emulgatoren werden in den zitierten Patenten nicht als vorteilhaft angesehen. Ferner wird als bevorzugter Emulgator Sorbitansesquioleat verwendet (Beispiele). Nachteilig ist, dass dieser leicht zur Oxidation neigt und zur Destabilisierung von weiteren Inhaltsstoffen der Emulsion führen kann (Peroxidbildung).

Die WO 0203927 beschreibt Aerosolzubereitungen, die Dialkylcarbonate als Ölkörper enthalten. Die Beispiele 3 und 6 im Vergleich zu V2 und V3 zeigen auf, dass diese Formeln zu einem trockenen Hautgefühl aluminiumchlorohydrathaltiger Rezepturen führen, wenn Di-n-Octylcarbonat als Öl verwendet wird. Esteröle, Dicaprylylether (V1, V3) werden daher nicht als vorteilhaft angesehen. Es wird erwähnt, dass Tenside (S. 6) und Emulgatoren (S. 8) sowie Wasser (S.6, Zeile 1) verwendet werden können. Dass die Zubereitungen in Form von Emulsionen vorliegen können, wird nicht offenbart; die genannten Beispiele beziehen sich nur auf wasserfreie Zubereitungen, was verständlich ist, da ein trockenes Hautgefühl in Gegenwart der Dialkylcarbonate angestrebt wird. Lediglich auf Seite 21, letzter Absatz wird offenbart, dass entsprechende Rezepturen insbesondere nach der Phaseninversionsmethode hergestellt werden können. Es ist hinlänglich bekannt, dass die typischerweise heiße W/O-Emulsionen (60-85°C) durch die Phaseninversion in feinteilige O/W-Emulsion (PIT-Emulsion) überführt werden. In der genannten Schrift wird nicht beschrieben, dass Esteröle, Ether, Triglyceride usw. als Ölkörper in W/O-Emulsionen zu vorteilhaft zu versprühenden Aerosolen führen.

Ferner sind auch sprühbare W/O-Emulsionen an sich bekannt, beispielsweise die in den Schriften DE-10162844-A1, DE-10162842-A1, DE-10162841-A1, DE-10162840-A1 oder DE-10048683-A1 offenbarten. Bei den hier beschriebenen Zubereitungen kann es sich zwar um Spray-Produkte handeln, allerdings fehlt jegliche Offenbarung zu möglichen Treibgasen, so dass derartige Zubereitungen als Pumpsprays aufzufassen sind, bei der das Produkt in eine Zerstäuberflasche abgefüllt wird und durch mechanisches Ausbringen zerstäubt wird. Ferner werden in den zitierten Druckschriften keine Angaben zu Verhältnissen des Treibgases zum Füllgut offenbart.

Der Stand der Technik konnte daher nicht den Weg zur vorliegenden Erfindung weisen.

Es war nach all diesem überraschend und nicht vorhersehbar, dass bei Raumtemperatur als Aerosole versprühbare W/O-Emulsionen, enthaltend
A. eine Fettphase, welche
   (a) mindestens 90 Gew.-% bei Raumtemperatur flüssige Ölkomponenten und
   (b) höchstens 4 Gew.-% an Substanzen, gewählt aus der Gruppe der C3- bis C4-Ester von C12- bis C18-Alkansäuren, C8- bis C12-Alkanole und Silikonöle,
   enthält,
B. 20 bis 85 Gew.-% - bezogen auf das Gesamtgewicht der Zubereitung - Wasser,
C. einen oder mehrere W/O-Emulgatoren,
D. einen oder mehrere Stabilisatoren, ausgewählt aus der Gruppe PEG-45/Dodecylglycolcopolymer, PEG-22/Dodecylglycolcopolymer und/oder Methoxy PEG-22/dodecylglycolcopolymer und
E. ein oder mehrere lipophile Treibgase
den Mängeln des Standes der Technik abhelfen würde.

Sofern im Rahmen dieser Schrift die Formulierung "bezogen auf das Gesamtgewicht der Zubereitung" verwendet wird, ist damit - sofern nichts anderes gesagt wird - das Gesamtgewicht des Füllguts (ohne Treibgas) gemeint.

Die erfindungsgemäßen Zubereitungen stellen in jeglicher Hinsicht überaus befriedigende Präparate dar. Es war insbesondere überraschend, dass die erfindungsgemäßen Zubereitungen außerordentlich stabil sind. Unter Stabilität ist im Sinne der vorliegenden Erfindung insbesondere zu verstehen, dass die Zubereitungen entweder die Aerosol-Behältnisse nicht schädigen bzw. korrodieren (unabhängig davon, ob die Zubereitungen selbst stabile oder instabile W/O-Emulsionen darstellen) oder dass die Zubereitungen nach Zusatz des Treibgases stabil bleiben oder beides.

Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische Eigenschaften, wie beispielsweise die Verteilbarkeit auf der Haut oder das Einzugsvermögen in die Haut, und zeichnen sich darüberhinaus durch eine überdurchschnittlich gute Hautpflege und einen angenehmen Kühleffekt aus.

Die erfindungsgemäßen Zubereitungen stellen hervorragende Grundlagen für Wirkstoffe dar, so dass die Zubereitungen in einer besonders bevorzugten Ausführungsform einen oder mehrere kosmetische Wirkstoffe enthalten.

Es bevorzugt im Sinne der vorliegenden Erfindung, wenn der W/O-Emulgator oder die W/O-Emulgatoren gewählt werden aus der Gruppe der Substanzen der allgemeinen Formel 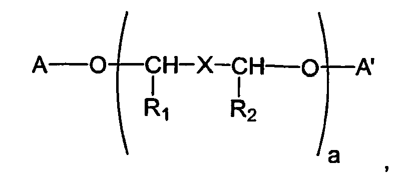 wobei
- A und A' gleiche oder verschiedene hydrophobe organische Reste darstellen,
- a eine Zahl von 1 bis 100, vorzugsweise 2 bis 60, insbesondere 5 bis 40 darstellt,
- X eine Einfachbindung oder die Gruppe 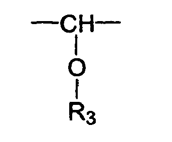
- darstellt,
- R₁ und R₂ unabhängig voneinander so gewählt werden H, Methyl, dass aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 bis 20 Kohlenstoffatomen.

Besonders bevorzugt ist es, wenn der W/O-Emulgator oder die W/O-Emulgatoren so gewählt werden, dass die Reste A und A' gewählt sind aus der Gruppe der verzweigten und unverzweigten, gesättigten und ungesättigten Alkyl- und Acylreste und Hydroxyacylreste mit 10 bis 30 Kohlenstoffatomen sowie ferner aus der Gruppe der über Esterfunktionen miteinander verbundenen Hydroxyacylgruppen, nach dem Schema: 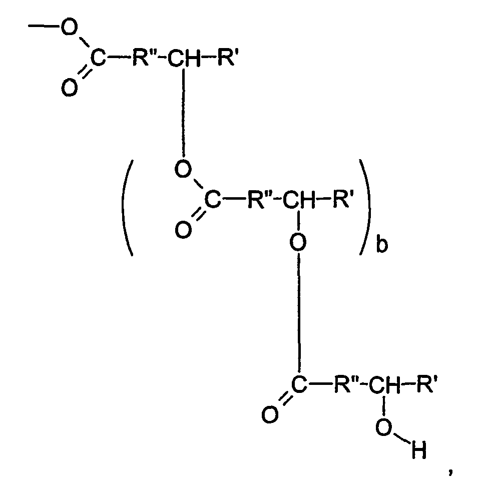 wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

Insbesondere bevorzugt ist es, wenn der oder die W/O-Emulgatoren gewählt werden aus der Gruppe PEG-30 Dipolyhydroxystearat, Decaglycerylheptaoleat, Polyglyceryl-3-Diisostearat, PEG-8 Distearat, Diglycerin Dipolyhydroxystearat, Glycerinisostearat, Sorbitanisostearat, Polyglyceryl-3 Methylglucosedistearat, Fettalkohole mit 8 bis 30 Kohlenstoffatomen, Oligo- oder Polyglycerinether, Cetyl Dimethicon Copolyole, Alkyl Methicon Copolyole, Alkyl Dimethicon Ethoxy Glucoside, W/O-Emulgatoren, die zusätzlich (poly-)ethoxyliert und/oder (poly-)propoxyliert sind, beispielsweise polyethoxyliertes hydrogeniertes oder nichthydrogeniertes Ricinusöl, ethoxyliertes Cholesterin, ethoxlierte Fettalkohole wie Steareth-2, ethoxlierte Fettsäuren wie PEG-2 Stearat, PEG-40 Sorbitanperisostearat.

Bevorzugt ist es, wenn der oder die W/O-Emulgatoren gewählt werden aus der Gruppe PEG-30 Dipolyhydroxystearat, Polyglyceryl-3-DÜsostearat (= Polyglycerin-3-DÜsostearat), Diglycerin Dipolyhydroxystearat, Glycerinisostearat, Cetyl PEG/PPG-10/1 Dimethicon, Sorbitanisostearat, Polyglyceryl-3 Methylglucosedistearat, Steareth-2, PEG-2 Stearat, Sorbitanstearat, Cetylalkohol, Stearylalkohol und/oder Cetearylalkohol.

Ganz besonders bevorzugt ist es, wenn Kombinationen der oben genannten W/O-Emulgatoren, insbesondere eine Kombination aus zwei Emulgatoren, eingesetzt werden

Der erfindungsgemäß verwendete W/O-Emulgator bzw. die erfindungsgemäß verwendeten W/O-Emulgatoren liegt bzw. liegen vorteilhaft in Konzentrationen von 0,5 bis 8 Gew.-% (bezogen auf das Gesamtgewicht der Zubereitung) vor, wobei es allerdings möglich und vorteilhaft ist, den Gehalt an Emulgatoren niedrig zu halten, etwa bis 5 Gew.-%, jeweils bezogen auf das Gesamtgewicht der Zusammensetzung. Es ist insbesondere vorteilhaft, die Konzentration an W/O-Emulgatoren (eine oder mehrere Verbindungen) aus dem Bereich von 1 Gew.-% bis 5 Gew.-% (bezogen auf das Gesamtgewicht der Zubereitung) zu wählen.

Ferner ist es vorteilhaft, die Emulgatoren so zu wählen, dass Kombinationen aus W/O-und O/W-Emulgatoren zum Einsatz kommen.

Als Stabilisator wird das PEG-45 /Dodecylglycolcopolymer und/oder das PEG-22 / Dodecylglycolcopolymer und/oder das Methoxy PEG-22/Dodecyl Glycol Copolymer verwendet.

Der Stabilisator bzw. die Stabilisatoren liegen vorteilhaft in Konzentrationen bis 8 Gew.-% vor, wobei es allerdings möglich und vorteilhaft ist, den Gehalt an Stabilisatoren niedrig zu halten, etwa bis 5 Gew.-%, jeweils bezogen auf das Gesamtgewicht der Zusammensetzung.

Es ist insbesondere dann vorteilhaft, Stabilisatoren zu wählen, wenn die erfindungsgemäßen Zubereitungen einen hohen Gehalt an destabilisierenden Substanzen, beispielsweise Parfümbestandteile, schwer zu emulgierende Wasser- oder Fettphasenbestandteile - wie beispielsweise Antitranspirantwirkstoffe - oder weitere Füllstoffe, enthalten sollen.

Es ist ganz besonders vorteilhaft, Stabilisatoren zu verwenden, wenn der pH-Wert der erfindungsgemäßen Zubereitungen im sauren Bereich liegt.

Es ist vorteilhaft im Sinne der vorliegenden Erfindung, das Gewichts-Verhältnis von W/O-Emulgatoren zu Stabilisatoren von 1:4 bis 4:1, bevorzugt ungefähr 1 : 1 zu wählen.

Bevorzugt ist es, wenn die Ölkomponente oder die Gesamtheit der Ölkomponenten gewählt wird aus der Gruppe der verzweigten und unverzweigten Kohlenwasserstoffe, der cyclischen oder linearen Silikonöle, der Dialkylether, der Dialkylcarbonate, der Gruppe der gesättigten oder ungesättigten, verzweigten Alkohole, sowie der Fettsäuretriglyceride, namentlich der synthetischen oder natürlichen Triglycerinester gesättigter und/oder ungesättigter, verzweigter und/oder unverzweigter Alkancarbonsäuren einer Kettenlänge von 8 bis 24, insbesondere 12 bis 18 C-Atomen, der Gruppe der Ester aus gesättigten und/oder ungesättigten, verzweigten und/oder unverzweigten Alkanmono oder Dicarbonsäuren einer Kettenlänge von 1 bis 44 C-Atomen und gesättigten und/oder ungesättigten, verzweigten und/oder unverzweigten Alkoholen oder Diolen einer Kettenlänge von 1 bis 44 C-Atomen, aus der Gruppe der Ester oder Diester aus aromatischen und/oder nicht aromatischen Carbonsäuren und gesättigten und/oder ungesättigten, verzweigten und/oder unverzweigten Alkoholen (einwertig oder mehrwertig) einer Kettenlänge von 1 bis 30 C-Atomen sofern die Ölkomponente oder die Gesamtheit der Ölkomponenten bei Raumtemperatur eine Flüssigkeit darstellen.

Besonders vorteilhafte Ölkomponenten im Sinne der vorliegenden Erfindung sind beispielsweise Dicaprylylcarbonat, Dicaprylylether, 2-Ethylhexylcocoat, Myristylmyristat, Octyldodecanol, Polydecen, Squalan, Triisostearin, Capryl-Caprinsäure-Triglycerid, Di-(2-Ethylhexyl)adipat, Ricinusöl, Avocadoöl, Macadamiaöl, Sojaöl, Jojobaöl. Auch Cetearyl Isononanoat und Cyclomethicon sind vorteilhaft im Sinne der vorliegenden Erfindung zu verwenden.

Wenn die erfindungsgemäßen Zubereitungen UV-Filtersubstanzen enthalten, ist es vorteilhaft, wenn die Ölphase Butylenglykol-Derivate (wie beispielsweise Butylenglykol Dicaprylat), Triglyceride (wie beispielsweise Capryl-Caprinsäure-Triglycerid [caprylic/capric triglycerid]), C₁₂-C₁₅ Alkylbenzoat und/oder Silikonöle enthält bzw. gänzlich aus solchen Ölen besteht.

Die Fettphase kann bis zu etwa 20 Gew.-% betragen, bezogen auf das Gesamtgewicht der Zubereitungen, wobei üblicherweise optimale Lipidgehalte im Bereich zwischen 5 und 15 Gew.-% gewählt werden. Gewünschtenfalls kann der Mindestlipidgehalt zwar 5 Gew.-% unterschreiten. Es ist aber von Vorteil, erfindungsgemäße Zubereitungen mit Gehalt von über 5 Gew.-% Lipide auszustatten, insbesondere dann, wenn fettlösliche oder fettdispergierbare Wirkstoffe wie beispielsweise Lichtschutzfilter, Wirkstoffe, Antioxidantien, Vitamine, Pigmente, Puder, Deo- und/oder Antitranspirantwirkstoffe in dem Fachmann bekannten Konzentrationen eingesetzt werden sollen.

Die Wassermenge kann bis zu etwa 85 Gew.-% betragen, bezogen auf das Gesamtgewicht der Zubereitungen, wobei üblicherweise optimale Wassergehalte im Bereich zwischen 50 und 80 Gew.-% gewählt werden. Gewünschtenfalls kann der Mindestwassergehalt zwar 50 Gew.-% unterschreiten. Es ist aber von größerem Vorteil, erfindungsgemäße Zubereitungen mit Gehalt von über 50 Gew.-% Wasser auszustatten insbesondere dann, wenn wasserlösliche oder wasserdispergierbare Stoffe wie Hautbefeuchtungsmittel, Deo- und Antitranspirantwirkstoffe, Polymere, Salze in dem Fachmann bekannten Konzentrationen eingesetzt werden sollen.

Als Hautbefeuchtungsmittel, Moisturizer, lassen sich vorteilhaft eine oder mehrere Substanzen aus der Gruppe: Glycerin, Chitosan, Fucogel, Milchsäure, Propylenglycol, Dipropylenglycol, Butylenglycol, Mannitol, Natriumpyrolidoncarbonsäure, Hyaluronsäure und deren Salze, Aminosäuren wie Glycin, Harnstoff, Natrium und Kaliumsalze verwenden. Als Salze werden bevorzugt ein- oder mehrwertige Kationen gewählt (z.B Natrium, Magnesium, Aluminium). Ferner vorteilhaft im Sinne der vorliegenden Erfindung kann auch Ethanol zur Wasserphase hinzugefügt werden, z. B. wenn ein Frische-Effekt bevorzugt ist oder die lipophile Phase der Komposition weiter verringert werden soll. Ferner kann Ethanol auch zur Wirkungsverstärkung benutzt werden (Deo Wirkung, AT-Wirkung, Verbesserung der Hautpflegeleistung, Lichtschutzleistung, Haarpflegeleistung).

In einer besonders vorteilhaften Ausführungsform enthalten die erfindungsgemäßen Zubereitungen einen oder mehrere der üblichen desodorierenden und/oder antitranspirant wirksamen Wirkstoffe, beispielsweise Geruchsüberdecker, wie die gängigen Parfümbestandteile, Geruchsabsorber, beispielsweise die in der Patentoffeniegeungschrift DE-P 40 09 347 beschriebenen Schichtsilikate, von diesen insbesondere Montmorillonit, Kaolinit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind 2,4,4'-Trichlor-2'hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-37 40 186, DE-39 38 140, DE-42 04 321, DE-42 29 707, DE-42 29 737, DE-42 37 081, DE- 43 09 372, DE-43 24 219 beschriebenen wirksamen Agenzien. Beispielsweise sind Glycerinester (Glycerinlaurat, -caprylat, -caprinat, Diglycerinmonocaprinat, Trigtycerinmonolaurat) einzeln oder in Kombination geeignete Wirkstoffe. Aber auch Glycerinether (Octoxyglycerin) oder Säuren wie 2-Butyloktansäure oder Sucroseester (Sucroselaurat) oder Cyclodextrine haben sich einzeln oder in Kombination bewährt. Ferner können auch Antiadhäsiva - beispielsweise die in DE 196 34 021, DE 196 43 585, DE 198 41 796, DE 198 41 795, DE 198 41 794, DE 197 18 777, DE 197 21 411 beschriebenen - vorteilhaft eingesetzt werden. Besonders vorteilhaft kann beispielsweise Chitosan als antiadhäsive Substanz eingesetzt werden. Wasserlösliche Polymere wie Chitosan, Cellulosen, Polyurethane, Carbomere, welche in wasserfreien Aerosolerzeugnissen des Standes der Technik wegen fehlender Wasserphase nicht formulierbar sind, können in den erfindungsgemäßen Zubereitungen vorteilhaft eingesetzt werden.

Die üblichen Antitranspiranswirkstoffe können ebenfalls vorteilhaft in den erfindungsgemäßen Zubereitungen verwendet werden, insbesondere Adstringentien, beispielsweise Aluminumchloride und/oder Aluminumchlorohydrate, Aluminium-Zirkonium Salze, Quats und dergleichen.

Die Konzentration der Wirkstoffe kann an sich nahezu beliebig gewählt werden, überschreitet jedoch in der Regel nicht ca. 80 Gew.-%, bezogen auf das Gesamtgewicht der Zubereitung.

Es können Zubereitungen aus fett- und wasserlöslichen Wirkstoffen eingesetzt werden, was bei Aerosolen des Standes der Technik wegen der fehlenden Wasserphase und Grenzfläche nicht möglich ist. Es zeigt sich, dass die Wirksamkeit der Zubereitungen im Vergleich zu Zubereitungen des Standes der Technik deutlich verbessert wird. Besonders vorteilhaft ist, dass Deo- und Antitranspirantwirkstoffe entsprechend ihrer Polarität in die Wasserphase, die Fettphase, an der Grenzfläche der Zubereitung eingesetzt werden können. Ferner ist es möglich, Aluminumchlorohydrat nicht nur als Pulver einzusetzen, sondern in seiner gelösten "wässrigen" Form.

Die Menge der Deo- und/oder Antitranspirantwirkstoffe in den Zubereitungen beträgt vorzugsweise 0,1 bis 60 Gew.-%, besonders bevorzugt 0,15 bis 50 Gew.-%, insbesondere 0,2 bis 40 Gew.-%, bezogen auf das Gesamtgewicht der Zubereitung.

Als Treigbas wird erfindungsgemäß bevorzugt Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Stickstoffoxide, Lachgas, 1,1,1,3 Tetrafluorethan, Heptafluoro-n-propan, Perfluorethan, Monochlordifluormethan, 1,1-Difluorethan einzeln oder in Kombination eingesetzt. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuß vorliegt.

Besonders bevorzugt sind Propan, Butan, iso-Butan bzw. Mischungen dieser Treibgase.

Die genannten Gase können im Sinne der vorliegenden Erfindung jeweils einzeln oder in beliebigen Mischungen zueinander verwendet werden.

Die Konzentration der Treibgase beträgt üblicherweise 10 bis 90 Gew.%, bevorzugt 40 bis 80 Gew.%, jeweils bezogen auf das Gesamtgewicht der Zubereitung.

Als Druckgasbehälter kommen Gefäße aus Metall (Aluminium, Weißblech), geschütztem bzw. nicht-splitterndem und splittemdem Glas oder Kunsstoff in Frage, bei deren Auswahl Druck- und Bruchfestigkeit, Korrosionsbeständigkeit, leichte Füllbarkeit als auch ästhetische Gesichtspunkte, Handlichkeit, Bedruckbarkeit etc. eine Rolle spielen. Spezielle Innenschutzlacke gewährleisten die Korrosionsbeständigkeit der Wasser-in-Öl Emulsion.

Die Zubereitung kann auch zusätzlich Ethanol enthalten. Ethanol ist zum Beispiel bevorzugt, wenn der Frischeeffekt, der durch den hohen Wassergehalt der erfindungsgemäßen Zubereitungen hervorgerufen wird, weiter gesteigert werden soll.

Die Fettphase der Zubereitung kann zusätzlich mit dem Fachmann bekannten Konsistenzgebern versehen sein (Wachse, Silica etc). Ferner kann auch die dispergierte Wasserphase Konsistenzgeber enthalten wie Cellulose, Carbomere, Chitosan etc. Besonders vorteilhaft ist Hydroxyethylcellulose und Chitosan. Nach der Applikation auf der Haut erhält man nach Verdunsten des Treibgases dann ein gelförmiges Produkt auf der Haut.

Ferner kann die Zubereitungen Korrosionsinhibitoren und Konservierungsstoffe, Antioxidantien, Chelatbildner enthalten.

Die Aerosoldose kann aus Aluminium, Zinn oder Weißblech bestehen.

Überraschend wurde gefunden, dass Zubereitungen mit den vorab beschriebenen Merkmalen zu versprühbaren Emulsionen führen. Vorteilhaft ist insbesondere, dass die versprühten Produkte sich auf der Haut durch ein angenehmes, nicht klebriges Hautgefühl auszeichnen.

Aufgrund des enthaltenden Wassers wird ein Frischegefühl nach der Anwendung festgestellt. Das Frischegefühl von marktgängigen Formulierungen wird hingegen üblicherweise durch Verwendung von Ethanol erzeugt.

Erfindungsgemäße Zubereitungen können aber auch Ethanol enthalten und ohne Verlust an Stabilität mit Ethanol formuliert werden. Emulsionen mit Ethanol sind häufig schwierig zu stabilisieren. Die Korrosionsbeständigkeit trotz einer Wasserphase im Aerosolbehältnis ist als Vorteil anzusehen. Ferner kann gezeigt werden, dass die Produkte eine ausgezeichnete Hautverträglichkeit aufweisen.
Ein weiterer Vorteil der erfindungsgemäßen Zubereitungen ist das ein Gewichts-Verhältnis von Füllgut zu Treibgas von beispielsweise 50/50 bis 80/20 (statt markttypisch 20/80) gewählt werden kann. Erfindungsgemäß sind daher Füllgut/Treibgasverhältnisse wählbar in Bereichen von 20/80 zu 80/20, bevorzugt im Bereich von 30/70 bis 50/50 wie beispielsweise 30/70, 40/60 oder 50/50. Die erfindungsgemäßen Zubereitungen ermöglichen demnach eine deutliche Reduktion flüchiger organischer Verbindungen im Vergleich zu klassischen Aerosol-Produkten, wodurch die Brennbarkeit der Produkte herabgesetzt bzw. sogar ganz unterdrückt wird. Ferner kann gezeigt werden, dass die Entflammbarkeit beim Versprühen des Produkts aufgrund des Wassergehalts bei Verwendung brennbarer Treibgase nicht auftritt, da der Wasseranteil dem entgegen wirkt. Als Füllgut wird die Summe aller Bestandteile ohne das Treibgas bezeichnet.

Darüberhinaus hat eine erfindungsgemäße Aerosoldose gleichen Volumens sozusagen mehr kosmetischen Inhalt als Aerosolprodukte des Standes der Technik. Dementsprechend können jetzt alternativ auch kleinere Aerosoldosen mit der erfindungsgemäßen W/O-Emulsion gewählt werden, die die gleiche Ergiebigkeit und Effektivität aufweisen wie wasserfreie, typische Produkte des Standes der Technik. Auf diese Weise kann daher bei gleicher Ergiebigkeit auch Aerosolpackmittel eingespart werden.

Erfindungsgemäße Emulsionen können auch Puderstoffe und/oder Füllstoffe enthalten. Als Puderstoffe werden beispielsweise Wismuthoxichlorid, titanisierter Glimmer, Siliciumdioxid (fumed silica), spherische Siliciumdioxid-Perlen, Tapiocastärke, Distärkephosphat, Polymethylmethacyrlat-Perlen, micronisiertes Teflon, Bornitrid, Acrylatpolymere, Aluminumsilicat, Aluminum-Stärke-Octenylsuccinat, Bentonit, Calciumsilicat, Cellulose, Kreide, Maisstärke, Glycerylstärke, Hectorit, hydrisiertes Silica, Kaolin, Magnesiumhydroxide, Magnesiumoxid, Magnesiumsilicate, Magnesiumtrisilicat, Maltodextrin, Montmorillonit, microcristalline Cellulose, Reisstärke, Silica, Talk, Mica, Titaniumdioxid, Zinklaurate, Zinkmyristat, Zinkneodecanoat, Zinkrosinat, Zinkstearat, Polyehtylen, Aluminiumoxid, Attapulgit, Calciumcarbonat, Calciumsilicat, Dextran, Kaolin, Lauroyl Lysin, Nylon, Silicasilylat, Seidenpuder, Serecit, Zinnoxid, Titaniumhydroxid, Trimagnesiumphosphat, Pigmente, die weder hauptsächlich UV-Filter- noch färbende Wirkung haben (wie z. B. Bornitrid etc.) und/oder Aerosile^{®} (CAS-Nr. 7631-86-9), Wallnußschalenpuder oder beliebige Mischungen eingesetzt werden.

Auch sog. Microspheres (spezielle Puder) sind Füllstoffe im Sinne der vorliegenden Erfindung. Microsheres sind beispielsweise von der Firma Kobo auf Basis Polymethylmethacrylat, Polyethylen, Ethylen/Acrylate-Copolymer, Nylon 12, Polyurethan, Silikon Resin und Silica erhältlich. Das Hautgefühl lipidischer Zubereitungen kann je nach Wahl der Micropsheres von soft, cremig, weich/naß, slippery zu hart/trocken eingestellt werden. Man nimmt an, dass bei Verwendung von Microspheres gewissermaßen kleine, runde Kugeln über die Hautoberfläche rollen und so die interessante Sensorik hervorrufen.

Ferner können auch entsprechende Microsphere-Komplexe von der Firma Kobo vorteilhaft als Füllstoffe eingesetzt werden. Hierbei handelt es sich um mit Isopropyltitantriisostearat versehene Mikrospheren, die ferner weiter modifiziert werden können.

Vorteilhafte Füllstoffe im Sinne der vorliegenden Erfindung sind auch Pigmente. Vorteilhaft sind beispielsweise Pigmente auf Basis von Silikaten (Talk, Mica, Sericit, Ultramarin), Metalloxiden (Eisen, Titan, Zirkon), Metallhydroxyiden (Eisen, Chrom), Compositen (Titanbehandeltes Mica), Füllstoffen (Stärke, Cellulosen) und dergleichen.

Ferner können die Pigmente modifiziert sein, wie z. B. aminosilikonbehandelte Pigmente und/oder mit Silan-, Methicon- oder Dimethicon behandelt.

Sollen die Zubereitungen beispielsweise als Körperspray zur Veränderung des optischen Aussehens der betreffenden Person benutzt werden (z. B. für die Füße, Oberarme, Beine, das Gesicht, den Nacken, das Gesäß, die Rückenpartie), so können besonders vorteilhaft Selbstbräuner (Dihydroxyaceton etc.) oder auch farbige Pigmente (Eisenoxide etc.) oder eine Kombination daraus eingesetzt werden. Nach Versprühen auf das entsprechende Körperareal entsteht auf diese Weise eine Farbvertiefung/-veränderung gegenüber der unbehandelten Haut. Bei Versprühen des Produkts auf die Beine kann der Eindruck eines getragenen Strumpfes bzw. einer Strumpfhose hervorgerufen werden, beim Versprühen auf die Arme kann der Eindruck eines langen Ärmels hervorgerufen weden, nackte Füsse können den Eindruck eines getragenen Schuhs hervorufen, beim Versprühen auf die Haare werden andere Farben als die des Naturhaars hervorgerufen usw.

Neben der dekorativen Veränderung der Haut und/oder des Haares oder der Selbstbräunung ist auch die Einarbeitung von Hautaufhellenden Stoffen in die erfindungsgemäße Zubereitung möglich, wie beispielsweise Dioic Acid oder Stoffenauf der Grundlage von Hydrochinon.
Auch die Inhibierung der Tyrosinase mit Substanzen wie Kojisäure, Ascorbinsäure und Azelainsäure sowie deren Derivaten ist geläufig und kann erfindungsgemäß eingesetzt werden.

Ein erfindungsgemäßes Aerosol ist für diese (dekorative) Anwendung vorteilhafter als eine klassische Emulsion, da das Verteilen einer treibgasfreien Emulsion (die z. B. einen Selbstbräuner, hautaufhellende Substanz oder farbige Pigmente enthält) über den ganzen Körper langwierig ist.

Es ist auch möglich, zusätzliche Substanzen zu verwenden, welche die Konsistenz der erfindungsgemäßen Zubereitungen modifizieren, beispielsweise Verdicker.

Eine erstaunliche Eigenschaft der vorliegenden Erfindung ist, dass erfindungsgemäße Zubereitungen sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrunde steht wie die Bekämpfung der oxidativen Beanspruchung der Haut. Es ist aber auch günstig, die erfindungsgemäßen Zubereitungen mit einem Gehalt an einem oder mehreren Antioxidantien zu versehen, wenn die Zubereitungen einem anderen Zwecke dienen sollen, z.B. als Desodorantien oder Sonnenschutzmittel.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden.

Eine erstaunliche Eigenschaft der erfindungsgemäße Zubereitungen ist, dass diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem

Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkemöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit^{®} und Neocerit^{®}.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestem von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw.

Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

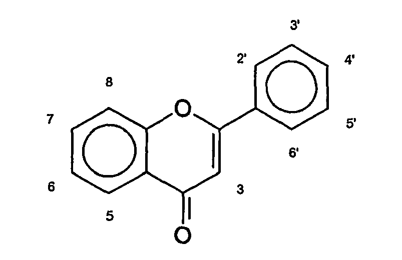

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt aus der Gruppe der Substanzen der generischen Strukturformel 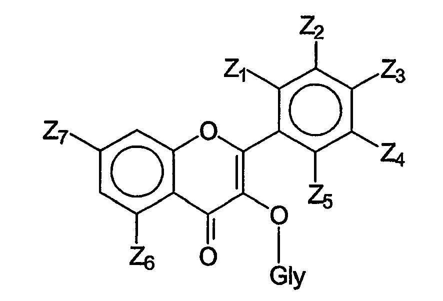 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 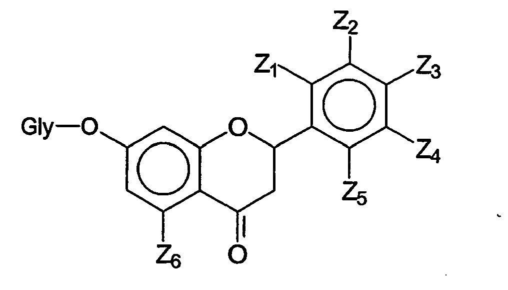 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 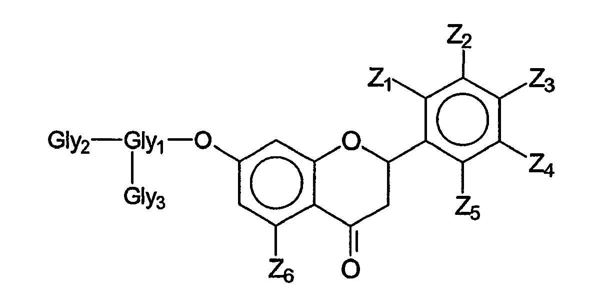 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struk tur

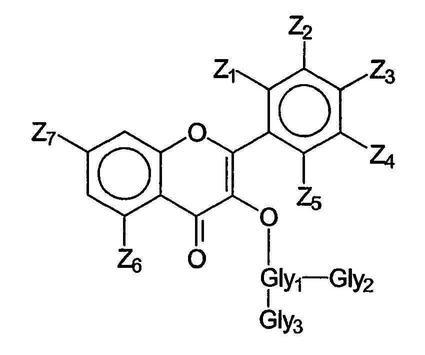

Besonders vorteilhaft werden die erfindungsgemäßen Flavonglycoside aus der Gruppe, welche durch die folgende Struktur wiedergegeben werden: 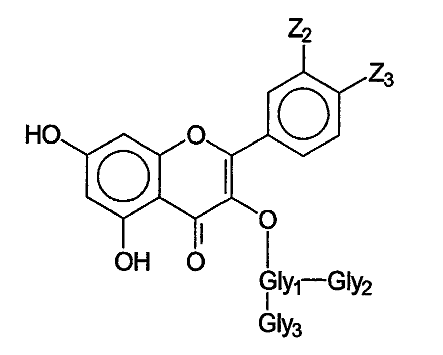 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-a-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, den oder die Wirkstoffe aus der Gruppe der Ubichinone und Plastochinone zu wählen.

Ubichinone zeichnen sich durch die Strukturformel 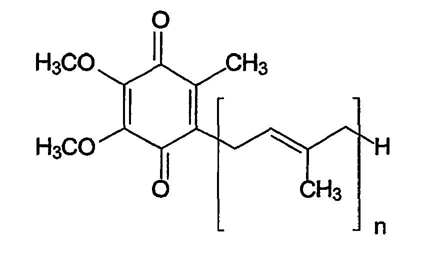 aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

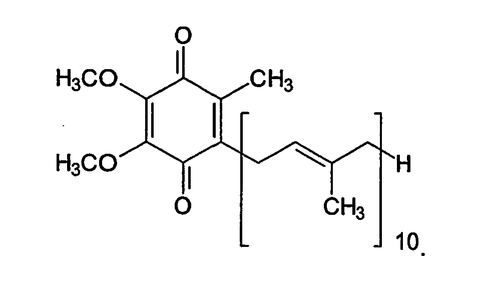

Plastochinone weisen die allgemeine Strukturformel 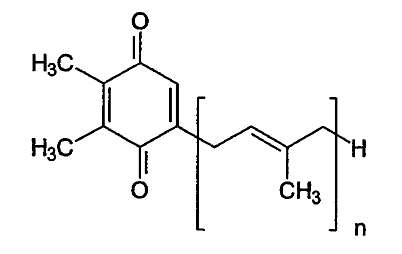 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate, Phsophokreatin sind bevorzugte Wirkstoffe im Sinne der vorliegenden Erfindung. Kreatin zeichnet sich durch folgende Struktur aus:

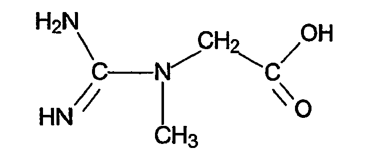

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Camitin [3-Hydroxy-4-(trimethylammonio)-buttersäurebetain]. Auch Acyl-Carnitine, welche gewählt aus der Gruppe der Substanzen der folgenden allgemeinen Strukturformel 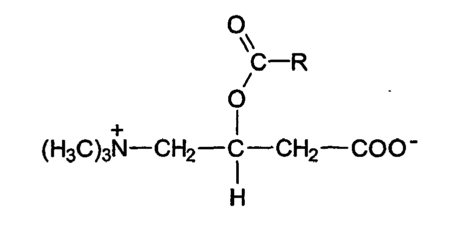 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Enantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Aminoguadin, Phytochelatin, Isoflavone (Genistein, Daidzein, Daidzin, Glycitin), Niacin, Tyrosinsulfat, Dioic Acid, Adenosin, Pyridoxin, Arginin, Vitamin K, Biotin und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Wirkstoffe können in den erfindungsgemäßen Zubereitungen in den Mengen von 0,0001 bis 25 Gew.%, vorzugsweise 0,001 bis 20 Gew.-%, insbesondere 0,01 bis 10 Gew.-% enthalten sein, jeweils bezogen auf das Gesamtgewicht der Zubereitungen.

Vorteilhaft ist es auch, die Wirkstoffe aus der Gruppe der rückfettenden Substanzen zu wählen, beispielsweise Purcellinöl, Eucerit^{®} und Neocerit^{®}.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen. Sie können auch als Sonnenschutzmittel dienen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die Zubereitungen gemäß der vorliegenden Erfindung können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten und welche insbesondere vorteilhaft auch frei von weiteren Ölkomponenten sein können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ- 303S | 3% Methicone | Tayca Corporation |
| MZ- 505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |
| Tioveil AQ 10PG | Alumina / Silica | Solaveil / Uniquema |
| Eusolex T-aqua | Wasser/Alumina/Natriummetaphosphat | Merck |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-sulfonsäure) bezeichnet wird. Benzol-1,4-di(2-oxo-3-bomylidenmethyl-10-sulfonsäure) hat die INCl-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel auszeichnen, 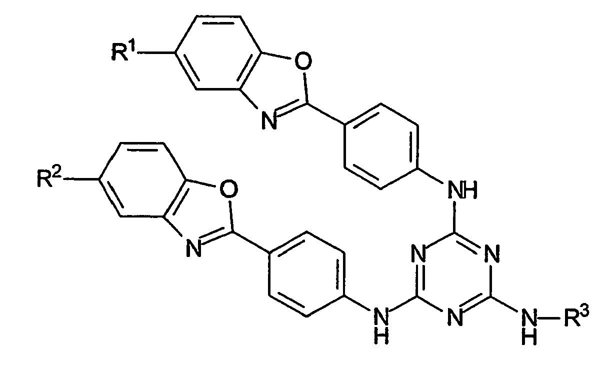 worin R¹, R² und R³ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste R¹ und R² gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn R³ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel 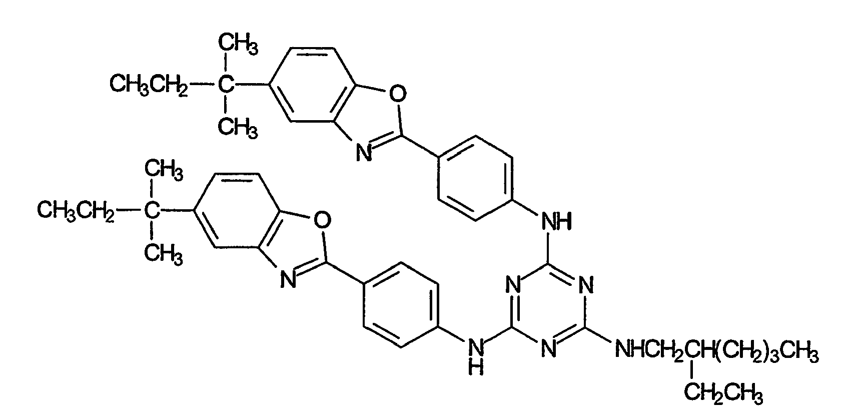 auszeichnet und bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus: 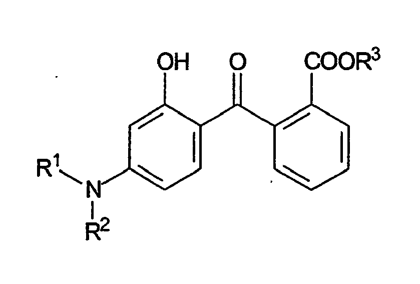 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 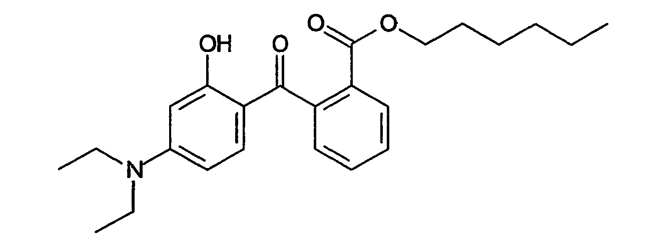 und unter dem Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCl: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCl: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z.B. unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

### Vorteilhafte wasserlösliche Filtersubstanzen sind z. B.:

Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A-und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP)

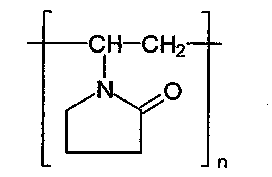

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Die Zubereitungen gemäß der vorliegenden Erfindung können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Zubereitung.

Es ist auch möglich und gegebenenfalls vorteilhaft, den erfindungsgemäßen Zubereitungen waschaktive Tenside zuzufügen. Erfindungsgemäße wäßrige kosmetische Reinigungsmittel oder für die wäßrige Reinigung bestimmte wasserarme oder wasserfreie Reinigungsmittelkonzentrate können kationische, anionische, nichtionische und/oder amphotere Tenside enthalten, beispielsweiseherkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaïne, Fettsäurealkanolamide, Polyglycolether-Derivate.

Kosmetische Zubereitungen, die kosmetische Reinigungszubereitungen für die Haut darstellen, können in flüssiger oder halbfester Form vorliegen, beispielsweise als Gele. Sie enthalten vorzugsweise mindestens eine anionische, kationische, nicht-ionische oder amphotere oberflächenaktive Substanz oder Gemische daraus, gegebenenfalls Elektrolyte und Hilfsmittel, wie sie üblicherweise dafür verwendet werden. Die oberflächenaktive Substanz kann bevorzugt in einer Konzentration zwischen 1 und 30 Gew.-% in den Reinigungszubereitungen vorliegen, bezogen auf das Gesamtgewicht der Zubereitungen.

Kosmetische Zubereitungen, die ein Shampoonierungsmittel darstellen, enthalten vorzugsweise mindestens eine anionische, nicht-ionische oder amphotere oberflächenaktive Substanz oder Gemische daraus, gegebenenfalls Elektrolyte und Hilfsmittel, wie sie üblicherweise dafür verwendet werden. Die oberflächenaktive Substanz kann bevorzugt in einer Konzentration zwischen 1 und 50 Gew.-% in den Reinigungszubereitungen vorliegen, bezogen auf das Gesamtgewicht der Zubereitungen. Vorteilhaft sind beispielsweise Cetyltrimethylammoniumsalze zu verwenden.

Die erfindungsgemäßen für die Reinigung des Haares oder der Haut vorgesehenen Zubereitungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die erfindungsgemäßen Zubereitungen haben erstaunlicher Weise sehr gute Pflegeeffekte und wirken in hohem Maße regenerierend in bezug auf den allgemeinen Hautzustand. Insbesondere wirken die erfindungsgemäßen Zubereitungen hautglättend, vermindern das Trockenheitsgefühl der Haut und machen die Haut geschmeidig.

Sollen die erfindungsgemäßen Zubereitungen zur Haarpflege eingesetzt werden, können sie die üblichen Bestandteile enthalten, üblicherweise zum Beispiel filmbildende Polymere. Von solchen Polymeren mit wenigstens teilweise quaternisierten Stickstoffgruppen (im folgenden "Filmbildner" genannt), eigenen sich bevorzugt solche, welche gewählt werden aus der Gruppe der Substanzen, welche nach der INCl-Nomenklatur (International Nomenclature Cosmetic Ingredient) den Namen "Polyquaternium" tragen, beispielsweise:

| | |
|---|---|
| Polyquaternium-2 | (Chemical Abstracts-Nr. 63451-27-4, z.B. Mirapol® A-15) |
| Polyquaternium-5 | (Copolymeres aus dem Acrylamid und dem β-Methacryloxyethyltrimethylammoniummethosulfat, CAS-Nr. 26006-22-4) |
| Polyquaternium-6 | (Homopolymer des N,N-Dimethyl-N-2-propenyl-2-propen-1-aminiumchlorids, CAS-Nr. 26062-79-3, z.B. Merquat® 100 |
| Polyquaternium-7 | N,N-Dimethyl-N-2-propenyl-2-propen-1-aminiumchlorid, Polymeres mit 2-Propenamid, CAS-Nr. 26590-05-6, z.B. Merquat® S |
| Polyquaternium-10 | Quaternäres Ammoniumsalz der Hydroxyethylcellulose, CAS-Nr. 53568-66-4, 55353-19-0, 54351-50-7, 68610-92-4, 81859-24-7, z.B. Celquat® SC-230M, |
| Polyquaternium-11 | Vinylpyrrolidon/dimethylaminoethyl-Methacrylat-Copolymer/Diethylsulfat-Reaktionsprodukt, CAS-Nr. 53633-54-8, z.B. Gafquat® 755N |
| Polyquaternium-16 | Vinylpyrrolidon/vinylimidazoliniummethochlorid-Copolymer, CAS-Nr. 29297-55-0, z.B. Luviquat® HM 552 |
| Polyquaternium-17 | CAS-Nr. 90624-75-2, z.B. Mirapol® AD-1 |
| Polyquaternium-19 | Quaternisierter wasserlöslicher Polyvinylalkohol |
| Polyquaternium-20 | in Wasser dispergierbarer quaternisierter Polyvinyloctadecylether |
| Polyquaternium-21 | Polysiloxan-polydimethyl-dimethylammoniumacetat-Copolymeres, z.B. Abil® B 9905 |
| Polyquaternium-22 | Dimethyldiallylammoniumchlorid/Acrylsäure-Copolymer, CAS-Nr. 53694-7-0, z.B. Merquat® 280 |
| Polyquaternium-24 | Polymeres quaternäres Ammoniumsalz der Hydroxyethylcellulose, Reaktionsprodukt mit einem mit Lauryldimethylammonium substituierten Epoxid, CAS-Nr. 107987-23-5, z.B. Quatrisoft® LM-200 |
| Polyquaternium-28 | Vinylpyrrolidon/Methacrylamidopropyltrimethylammoniumchlorid-Copolymer, z.B. Gafquat® HS-100 |
| Polyquaternium-29 | z.B. Lexquat® CH |
| Polyquaternium-31 | CAS-Nr. 136505-02-7, z.B. Hypan® QT 100 |
| Polyquaternium-32 | N,N,N-trimethyl-2-[(2-methyl-1-oxo-2-propenyl)oxy]-Ethanaminium-chlorid, polymer mit 2-Propenamid, CAS-Nr. 35429-19-7 |
| Polyquaternium-37 | CAS-Nr. 26161-33-1 |

Cetyltrimethylamoniumsalze wie CTAB, CTAC.

Vorteilhaft enthalten erfindungsgemäße Zubereitungen zur Haarpflege 0,01 bis 5 Gew.-% eines oder mehrerer Filmbildner, bevorzugt 0,1 bis 3 Gew.-%, insbesondere 0,2 bis 2 Gew.-%, jeweils bezogen auf das Gesamtgewicht der Zubereitungen. Derartige Ausführungsformen der erfindungsgemäßen Zubereitungen pflegen durch Umwelteinflüsse geschädigtes oder strapaziertes Haar bzw. beugen solchen Umwelteinflüssen vor. Ferner verleihen die erfindungsgemäßen Zubereitungen der Haartracht lockere Fülle und Festigkeit, ohne klebrig zu wirken.

Erfindungsgemäße kosmetische Zubereitungen zur Festigung und zum Styling der Haare enthalten üblicherweise Filmbildner, wie sie normalerweise in solchen Zubereitungen verwendet werden, wobei die Gesamtmenge der Filmbildnersubstanzen z. B. zwischen 0,5 und 20 Gew.-% beträgt, bezogen auf das Gesamtgewicht der Zubereitungen.

Erfindungsgemäß können als günstige Filmbildner alle für kosmetische und/oder dermatologische Anwendungen geeigneten oder gebräuchlichen Filmbildner verwendet werden.

Vorteilhaft werden der oder die Filmbildner gewählt aus der Gruppe der wasserlöslichen oder wasserdispergierbaren Polyurethane, Polyharnstoffe, Silikonharze und/oder Polyester sowie der nichtionischen, anionischen, amphoteren und/oder kationischen Polymere.

Vorteilhafte nichtionische Polymere, die in erfindungsgemäßen Zubereitungen alleine oder im Gemisch, vorzugsweise auch mit anionischen und/oder amphoteren und/oder zwitterionischen Polymeren enthalten sein können, sind Vinylpyrrolidon-Homo- oder Copolymerisate. Hierzu gehören beispielweise Polyvinylpyrrolidon, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat, Polysiloxane und dergleichen mehr.

Vorteilhafte anionische Polymere sind beispielsweise Vinylacetat/Crotonsäure-, Vinylacetat/Acrylat- und/oder Vinylacetat/Vinylneodecanoat/Crotonsäure-Copolymere, Natriumacrylat/Vinylalkohol-Copolymere, Natriumpolystyrolsulfonat, Ethylacrylat/N-tert.-Butylacrylamid/Acrylsäure-Copolymere, Vinylpyrrolidon/Vinylacetat/Itaconsäure-Copolymere, Acrylsäure/Acrylamid-Copolymere und/oder deren Natriumsalze, Homo- und/oder Copolymere von Acrylsäure und/oder Methacrylsäure und/oder deren Salze sowie Acrylat/Hydroxyacrylat-, Octylacrylamid/Acrylat- bzw. Methacrylsäurester und/oder Butylacrylat/N-Vinylpyrrolidon-Copolymere.

Weitere bevorzugte anionische Polymere sind Methylvinylether/Maleinsäure-Copolymere, die durch Hydrolyse von Vinylether/Maleinsäureanhydrid-Copolymeren entstehen. Diese Polymere können auch teilverestert sein (Ethyl, Isopropyl- bzw. Butylester).

Vorteilhafte amphotere Polymere, die in erfindungsgemäßen Zubereitungen alleine oder im Gemisch, vorzugsweise auch mit anionischen und/oder nichtionischen Polymeren enthalten sein können, sind Copolymerisate aus N-Octylacrylamid, (Meth)Acrylsäure und tert.-Butylaminoethylmethacrylat vom Typ "Amphomer", Copolymerisate aus Methacryloylethylbetain und Alkylmethacrylaten vom Typ "Yukaformer", Copolymerisate aus Carboxylgruppen oder Sulfongruppen enthaltenden Monomeren, z.B. (Meth)Acrylsäure und Itaconsäure, mit basischen, insbesondere Aminogruppen enthaltenden Monomeren wie z.B. Mono- bzw. Dialkylaminoalkyl(meth)acrylaten und/oder Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure, wobei diese Aufstellung selbstverständlich nicht limitierend sein soll.

Es ist gegebenenfalls vorteilhaft, die anionischen und amphoteren Polymere zur Verbesserung ihrer Wasserlöslichkeit bzw. ihrer Wasserdispergierbarkeit mit geeigneten Basen zu neutralisieren. Hierzu können beispielsweise Alkali- bzw. Erdalkalibasen, Ammoniak und/oder verschiedene Amine, wie z.B. Triethanolamin, Triisopropanolamin, Aminomethyl-propanol und/oder Aminomethylpropandiol, eingesetzt werden. Die Neutralisation kann je nach Anwendungszweck teilweise oder vollständig erfolgen.

Vorteilhafte kationische Polymere sind beispielsweise VinylpyrrolidonlVinylimidazoliummethochlorid-Copolymere, quaternisierte Vinylpyrrolidon/Dialkylaminoalkylmethacrylat-Copolymere, kationische Cellulose-Derivate, wie z.B. Hydroxyethylcellulose/Dimethylalkylammoniumchlorid-Copolymere sowie Terpolymere aus Vinylcaprolactam/Vinylpyrrolidon mit Dimethylaminoethylmethacrylat bzw. Vinylimmidazoliniummethochlorid und Acrylamidocopolymere.
Es ist auch vorteilhaft, Filmbildner auf natürlicher Basis, z.B. Chitosan und dessen Derivate, einzusetzen, insbesondere im Gemisch mit synthetischen Polymeren.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Entsprechend können die erfindungsgemäßen Zubereitungen, je nach ihrem Aufbau, beispielsweise verwendet werden als Hautschutzemulsion, Reinigungsmilch, Sonnenschutzlotion, Nährlotion, Tages- oder Nachtemulsion, Stylingemulsion usw.

Die erfindungsgemäßen Zubereitungen tragen ferner in vorzüglicher Weise zur Hautglättung bei, insbesondere, wenn sie mit einer oder mehreren Substanzen versehen sind, die die Hautglättung fördern.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeginet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Hormone, Steroide, Vitamine usw.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Viruzide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, Medikamente, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel.

Insbesondere vorteilhaft werden Gemische der vorstehend genannten Lösungsmittel verwendet.

Als weitere Bestandteile können verwendet werden Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeginet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Hormone, Steroide, Vitamine usw.

Die nachfolgenden Beispiele sollen die Erfindung erläutern, ohne sie einzuschränken. Alle Mengenangaben, Prozentangaben oder Teile beziehen sich, soweit nicht anders angegeben, auf das Gewicht, insbesondere auf das Gesamtgewicht der Zubereitungen oder der jeweiligen Mischungen.
Die Füllgut/Treibgasverhältnisse in den Beispiele können neben den angegeben Werten auch bevorzugt 30/70, 40/60 oder 50/50 betragen.

### Beispiel 1

### AT Aerosol

| Füllgut | Gew.-% |
|---|---|
| PEG-45/Dodecyl Glycol Copolymer | 1,60 |
| Diglycerin Dipolyhydroxystearat | 1,60 |
| Dicaprylylcarbonat | 10,00 |
| Aluminiumchlorohydrat | 20,00 |
| Parfüm | q.s. |
| Wasser | 66.0 |
| Abfüllverhältnis Füllgut/Treibgas: 50:50 (Gew%) | |
| Treibgas: Propan/Butan: 2.7 | |

### Beispiel 2

### AT Aerosol

| Füllgut | Gew.-% |
|---|---|
| PEG-45/Dodecyl Glycol Copolymer | 1,60 |
| Glyceryl Isostearat | 1,60 |
| Dicaprylylcarbonat | 10,00 |
| Aluminiumchlorohydrat | 20,00 |
| Parfüm | q.s. |
| Wasser | 66,00 |
| Abfüllverhältnis Füllgut/Treibgas: 50:50 (Gew%) | |
| Treibgas: Propan/Butan: 2.7 | |

### Beispiel 3

### AT Aerosol

| Füllgut | Gew.-% |
|---|---|
| PEG-45/Dodecyl Glycol Copolymer | 1,60 |
| Polyglyceryl-3 Diisostearat | 1,60 |
| Dicaprylylcarbonat | 10,00 |
| Caprlic/ Capric Triglycerid | 2,00 |
| Dicaprylylether | 2,00 |
| Aluminiumchlorohydrat | 20,00 |
| Parfüm, | q.s. |
| Wasser | 66,00 |
| Abfüllverhältnis Füllgut/Treibgas: 50:50 (Gew%) | |
| Treibgas: Propan/Butan: 2.7 | |

### Beispiel 4

### AT Aerosol

| Füllgut | Gew.-% |
|---|---|
| PEG-45/Dodecyl Glycol Copolymer | 1,60 |
| Polyglyceryl-3 Diisostearat | 1,60 |
| Dicaprylylcarbonat | 6,00 |
| Caprlic/ Capric Triglycerid | 2,00 |
| Dicaprylylether | 2,00 |
| Aluminiumchlorohydrat | 20,00 |
| Parfüm | q.s. |
| Wasser | 66,00 |
| Abfüllverhältnis Füllgut/Treibgas: 50:50 (Gew%) | |
| Treibgas: Propan/Butan: 2.7 | |

### Beispiel 5

### AT Aerosol

| Füllgut | Gew.-% |
|---|---|
| PEG-45/Dodecyl Glycol Copolymer | 1,60 |
| Polyglyceryl-3 Diisostearat | 1,60 |
| Dicaprylylcarbonat | 5,00 |
| Caprlic/ Capric Triglycerid | 2,00 |
| Octyldodecanol | 0,50 |
| Dicaprylylether | 1,50 |
| Aluminiumchlorohydrat | 30,00 |
| Parfüm | q.s. |
| Wasser | 57,00 |
| Abfüllverhältnis Füllgut/Treibgas: 50:50 (Gew%) | |
| Treibgas: Propan/Butan: 2.7 | |

### Beispiel 5a

### AT Aerosol

| Füllgut | Gew.-% |
|---|---|
| PEG-45/Dodecyl Glycol Copolymer | 1,60 |
| Polyglyceryl-3 Diisostearat | 1,60 |
| Dicaprylylcarbonat | 5,00 |
| Caprlic/ Capric Triglycerid | 2,00 |
| Octyldodecanol | 0,50 |
| Dicaprylylether | 1,50 |
| Aluminiumchlorohydrat | 30,00 |
| Parfüm | q.s. |
| Wasser | 57,00 |
| Abfüllverhältnis Füllgut/Treibgas: 30:70 (Gew%) | |
| Treibgas: Propan/Butan: 2.7 | |

### Beispiel 5b

### AT Aerosol

| Füllgut | Gew.-% |
|---|---|
| PEG-45/Dodecyl Glycol Copolymer | 1,60 |
| Polyglyceryl-3 Diisostearat | 1,60 |
| Dicaprylylcarbonat | 5,00 |
| Caprlic/ Capric Triglycerid | 2,00 |
| Octyldodecanol | 0,50 |
| Dicaprylylether | 1,50 |
| Aluminiumchlorohydrat | 30,00 |
| Parfüm | q.s. |
| Wasser | 57,00 |
| Abfüllverhältnis Füllgut/Treibgas: 40:60 (Gew%) | |
| Treibgas: Propan/Butan: 2.7 | |

### Beispiel 6

### Aerosol

| Füllgut | Gew.-% |
|---|---|
| PEG-45/Dodecyl Glycol Copolymer | 1,60 |
| Polyglycerin-3 Diisostearat | 1,60 |
| Cetearyl Isononanoat | 10,00 |
| Vitamin E acetat | 0.50 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 7

### Aerosol

| Füllgut | Gew.-% |
|---|---|
| PEG-45/Dodecyl Glycol Copolymer | 1,60 |
| Polyglycerin-3 Diisostearat | 1,60 |
| Dicaprylylether | 10,00 |
| Q-10 | 0.30 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 8

### Aerosol

| Füllgut | Gew.-% |
|---|---|
| PEG-45/Dodecyl Glycol Copolymer | 1,60 |
| Polyglycerin-3 Diisostearat | 1,60 |
| Caprylic/capric Triglycerid | 10,00 |
| Carnetin; Q10 (1:1) | 1,00 |
| Glycerin | 4,80 |
| Parfüm, Wasser | ad 100,00 |
| Abfüllverhältnis FüllgutlTreibgas: 50:50 (Gew%) | |
| Treibgas: Propan/Butan: 2.7 | |

### Beispiel 9

### Aerosol

| Füllgut | Gew.-% |
|---|---|
| PEG-45/Dodecyl Glycol Copolymer | 1,60 |
| Polyglycerin-3 Diisostearat | 1,60 |
| Octyldodecanol | 10,00 |
| Liponsäure, Biotin (1:1) | 1,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 10

### Aerosol

| Füllgut | Gew.-% |
|---|---|
| PEG-45/Dodecyl Glycol Copolymer | 1,60 |
| Polyglycerin-3 Diisostearat | 1,60 |
| Cyclomethicon | 10,00 |
| Cystein | 1.00 |
| Glycerin | 4,80 |
| Parfüm, Wasser | ad 100,00 |
| Abfüllverhältnis Füllgut/Treibgas: 30:70 (Gew%) | |
| Treibgas: Propan/Butan: 2.7 | |

### Beispiel 11

### Aerosol

| Füllgut | Gew.-% |
|---|---|
| PEG-45/Dodecyl Glycol Copolymer | 1,60 |
| Polyglycerin-3 Diisostearat | 1,60 |
| Polydecen | 10,00 |
| Vitamin C | 3.00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 12

### Aerosol

| Füllgut | Gew.-% |
|---|---|
| PEG-45/Dodecyl Glycol Copolymer | 1,60 |
| Polyglycerin-3 Diisostearat | 1,60 |
| Di-(2-Ethylhexyl)adipat | 10,00 |
| Salze (Natrium, Calcium, Magnesium) | 5,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 13

### Aerosol

| Füllgut | Gew.-% |
|---|---|
| PEG-45/Dodecyl Glycol Copolymer | 1,60 |
| Polyglycerin-3 Diisostearat | 1,60 |
| Avocadoöl, Jojobaöl (1:1) | 10,00 |
| AGR, Q-10 (1:1) | 1,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 14

### Aerosol mit Ethanol

| Füllgut | Gew.-% |
|---|---|
| PEG-45/Dodecyl Glycol Copolymer | 1,60 |
| Glycerinisostearat | 1,60 |
| Cetearyl Isononanoat | 10,00 |
| Carnitin (Acylcarnitin) | 0,30 |
| Glycerin | 4,80 |
| Ethanol | 5,00 |
| Parfüm, Wasser | ad 100,00 |
| Abfüllverhältnis Füllgut/Treibgas: 50:50 (Gew%) | |
| Treibgas: PropanButan: 2.7 | |

### Beispiel 15

### Aerosol

| Füllgut | Gew.-% |
|---|---|
| PEG-45/Dodecyl Glycol Copolymer | 1,60 |
| Sorbitanisostearat | 1,60 |
| Cetearyl Isononanoat | 10,00 |
| Dioic Acid | 0.50 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 16

### Aerosol

| Füllgut | Gew.-% |
|---|---|
| PEG-45/Dodecyl Glycol Copolymer | 1,60 |
| Diglycerinisostearat | 1,60 |
| Dicaprylylether | 10,00 |
| Isoflavon | 0,50 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 17

### Aerosol

| Füllgut | Gew.-% |
|---|---|
| PEG-45/Dodecyl Glycol Copolymer | 1,60 |
| PEG-30 Dipolyhydroxystearat | 1,60 |
| Caprylic/capric Triglycerid | 10,00 |
| Chitosan | 0,30 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 19

### Aerosol

| Füllgut | Gew.-% |
|---|---|
| PEG-45/Dodecyl Glycol Copolymer | 1,60 |
| Cetyldimethicon Copolyol | 1,60 |
| Octyldodecanol | 10,00 |
| Biotin | 1,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 20

### Aerosol

| Füllgut | Gew.-% |
|---|---|
| PEG-45/Dodecyl Glycol Copolymer | 1,60 |
| Steareth-2 (bzw. PEG-2 Stearat) | 1,60 |
| C12-C15 Alkyl Benzoat | 10,00 |
| Titandioxid (bzw Zinkoxid) | 2,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 21

### Aerosol

| Füllgut | Gew.-% |
|---|---|
| PEG-45/Dodecyl Glycol Copolymer | 1,60 |
| Diglycerin Dipolyhydroxystearat | 1,60 |
| Cetearyl lsononanoat; Butylenglycol Dicapry- | 12,00 |
| lat; Caprylic/Capric Triglycerid (1:1:1) | |
| Bis-Ethylhexyloxyphenol Methoxyphenyl Tria- | 4,00 |
| zin; Ethylhexyltriazon (5:3) | |
| Glycerin | 4,80 |
| PVP/Hexadecen Copolymer | 0.20 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 22

### Aerosol

| Füllgut | Gew.-% |
|---|---|
| PEG-45/Dodecyl Glycol Copolymer | 1,60 |
| Polyglyceryl-3 Methylglucosedistearat | 1,60 |
| Phenylbenzimidazolsulfonsäure | 2.00 |
| Natronlauge | 0.55 |
| Glycerin | 4,80 |
| Butylenglycol Dicaprylat | 10,00 |
| Octyltriazon | 2,00 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 23

### Aerosol

| Füllgut | Gew.-% |
|---|---|
| PEG-4.5/Dodecyl Glycol Copolymer | 1,60 |
| Polyglycerin-3 Diisostearat | 1,60 |
| Cetearyl Isononanoat | 10,00 |
| PVPNA Copolymer | 8,00 |
| Glycerin | 4,80 |
| Ethanol | 5,00 |
| Hydroxyethyl Cetyldimonium Phosphat | 0,1 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 24

### Aerosol

| Füllgut | Gew.-% |
|---|---|
| PEG-45/Dodecyl Glycol Copolymer | 1,60 |
| Polyglycerin-3 Diisostearat | 1,60 |
| Cetearyl Isononanoat | 10,00 |
| PVPNA Copolymer | 8,00 |
| Glycerin | 4,80 |
| Ethanol | 5,00 |
| Polyquaternium-16 | 1.0 |
| Parfüm, Wasser | ad 100,00 |
| Abfüllverhältnis Füllgut/Treibgas: 40:60 (Gew%) | |
| Treibgas: Propan/Butan: 2.7 | |

### Beispiel 25

### AT Aerosol

| Füllgut | Gew.-% |
|---|---|
| PEG-45/Dodecyl Glycol Copolymer | 1,60 |
| Polyglycerin-3 Diisostearat | 1,60 |
| Cetearyl Isononanoat | 10,00 |
| Aluminiumchlorphydrat | 20,00 |
| Glycerin | 4,80 |
| Parfüm, Wasser | ad 100,00 |
| Abfüllverhältnis Füllgut/Treibgas: 30:70 (Gew%) | |
| Treibgas: Propan/Butan: 2.7 | |

### Beispiel 26

### AT Aerosol

| Füllgut | Gew.-% |
|---|---|
| PEG-45/Dodecyl Glycol Copolymer | 1,60 |
| Polyglycerin-3 Diisostearat | 1,60 |
| Dicaprylylether | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 27

### AT Aerosol

| Füllgut | Gew.-% |
|---|---|
| PEG-45/Dodecyl Glycol Copolymer | 1,60 |
| Polyglycerin-3 Diisostearat | 1,60 |
| Caprylic/capric Triglycerid | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 28

### AT Aerosol

| Füllgut | Gew.-% |
|---|---|
| PEG-45/Dodecyl Glycol Copolymer | 1,60 |
| Polyglycerin-3 Diisostearat | 1,60 |
| Octyldodecanol | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 29

### AT Aerosol

| Füllgut | Gew.-% |
|---|---|
| PEG-45/Dodecyl Glycol Copolymer | 1,60 |
| Polyglycerin-3 Diisostearat | 1,60 |
| Cyclomethicon | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 30

### AT Aerosol

| Füllgut | Gew.-% |
|---|---|
| PEG-45/Dodecyl Glycol Copolymer | 1,60 |
| Polyglycerin-3 Diisostearat | 1,60 |
| Polydecen | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 31

### AT Aerosol

| Füllgut | Gew.-% |
|---|---|
| PEG-45/Dodecyl Glycol Copolymer | 1,60 |
| Polyglycerin-3 Diisostearat | 1,60 |
| Di-(2-Ethylhexyl)adipat | 10,00 |
| Aluminiumchlorohydrat | 20,00 |
| Glycerin | 4,80 |
| Parfüm, Wasser | ad 100,00 |
| Abfüllverhältnis FüIIgut/Treibgas: 50:50 (Gew%) | |
| Treibgas: Propan/Butan: 2.7 | |

### Beispiel 32

### AT Aerosol

| Füllgut | Gew.-% |
|---|---|
| PEG-45/Dodecyl Glycol Copolymer | 1,60 |
| Polyglycerin-3 Diisostearat | 1,60 |
| Avocadoöl, Jojobaöl (1:1) | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 33

### AT Aerosol mit Ethanol

| Füllgut | Gew.-% |
|---|---|
| PEG-45/Dodecyl Glycol Copolymer | 1,60 |
| Polyglycerin-3 Diisostearat | 1,60 |
| Cetearyl lsononanoat | 10,00 |
| Aluminiumchlorohydrat | 20,00 |
| Glycerin | 4,80 |
| Ethanol | 5,00 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 34

### Deo Aerosol

| Füllgut | Gew.-% |
|---|---|
| PEG-45/Dodecyl Glycol Copolymer | 1,60 |
| Polyglycerin-3 Diisostearat | 1,60 |
| Cetearyl Isononanoat | 10,00 |
| Glycerinmonolaurat | 1,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 35

### Deo Aerosol

| Füllgut | Gew.-% |
|---|---|
| PEG-45/Dodecyl Glycol Copolymer | 1,60 |
| Polyglycerin-3 Diisostearat | 1,60 |
| Dicaprylylether | 10,00 |
| Diglycerinmonocaprinat | 1,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 36

### Deo Aerosol mit Antiadhäsiva

| Füllgut | Gew.-% |
|---|---|
| PEG-45/Dodecyl Glycol Copolymer | 1,60 |
| Polyglycerin-3 Diisostearat | 1,60 |
| Caprylic/capric Triglycerid | 10,00 |
| Chitosan | 0,30 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 37

### AT Aerosol

| Füllgut | Gew.-% |
|---|---|
| PEG-45/Dodecyl Glycol Copolymer | 1,60 |
| Polyglycerin-3 Diisostearat | 1,60 |
| Octyldodecanol | 10,00 |
| Octoxyglycerin, Glycerinmonocaprinat (1:1) | 1,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 38

### Deo Aerosol

| Füllgut | Gew.-% |
|---|---|
| PEG-45/Dodecyl Glycol Copolymer | 1,60 |
| Polyglycerin-3 Diisostearat | 1,60 |
| Cyclomethicon | 10,00 |
| Sucroselaurat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 39

### Deo Aerosol

| Füllgut | Gew.-% |
|---|---|
| PEG-45/Dodecyl Glycol Copolymer | 1,60 |
| Polyglycerin-3 Diisostearat | 1,60 |
| Polydecen | 10,00 |
| 2-Butyloktansäure | 1,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 40

### Deo Aerosol

| Füllgut | Gew.-% |
|---|---|
| PEG-45/Dodecyl Glycol Copolymer | 1,60 |
| Polyglycerin-3 Diisostearat | 1,60 |
| Di-(2-Ethylhexyl)adipat | 10,00 |
| Triclosan | 1,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 41

### Deo Aerosol mit Ethanol

| Füllgut | Gew.-% |
|---|---|
| PEG-45/Dodecyl Glycol Copolymer | 1,60 |
| Polyglycerin-3 Diisostearat | 1,60 |
| Avocadoöl, Jojobaöl (1:1) | 10,00 |
| Glycerinmonolaurat | 1,00 |
| Glycerin | 4,80 |
| Ethanol | 5,00 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 42

### AT Aerosol

| Füllgut | Gew.-% |
|---|---|
| PEG-45/Dodecyl Glycol Copolymer | 1,60 |
| Glycerinisostearat | 1,60 |
| Cetearyl Isononanoat | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 43

### AT Aerosol

| Füllgut | Gew.-% |
|---|---|
| PEG-45/Dodecyl Glycol Copolymer | 1,60 |
| Sorbitanisostearat | 1,60 |
| Dicaprylylether | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 44

### AT Aerosol

| Füllgut | Gew.-% |
|---|---|
| PEG-45/Dodecyl Glycol Copolymer | 1,60 |
| PEG-30 Dipolyhydrooxystearat | 1,60 |
| Caprylic/capric Triglycerid | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 45

### AT Aerosol

| Füllgut | Gew.-% |
|---|---|
| PEG-45/Dodecyl Glycol Copolymer | 1,60 |
| Diglycerin Dipolyhydroxystearat | 1,60 |
| Octyldodecanol | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 46

### AT Aerosol

| Füllgut | Gew.-% |
|---|---|
| PEG-45/Dodecyl Glycol Copolymer | 1,60 |
| Steareth-2 (bzw. PEG-2 Stearat) | 1,60 |
| Cyclomethicon | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 47

### AT Aerosol

| Füllgut | Gew.-% |
|---|---|
| PEG-45/Dodecyl Glycol Copolymer | 1,60 |
| Polyglyceryl-3 Methylglucosedistearat | 1,60 |
| Polydecen | 10,00 |
| Aluminiumchlorohydrat | 20,00 |
| Glycerin | 4,80 |
| Parfüm, Wasser | ad 100,00 |
| Abfüllverhältnis Füllgut/Treibgas: 50:50 (Gew%) | |
| Treibgas: Propan/Butan: 2.7 | |

### Beispiel 48

Körperspray (Beine, Arm Gesicht, Fuß und/oder Rücken zur optischen Veränderung des Aussehens der Haut

| Füllgut | Gew.-% |
|---|---|
| PEG-45/Dodecyl Glycol Copolymer | 1,60 |
| Glyceryl lsostearat | 1,60 |
| Octyldodecanol | 10,00 |
| Farbpigment | 3,00 |
| Parfüm | q.s. |
| Wasser | Ad 100 |
| Farbpigment: | |
| Cl 77492: 0.3%; Cl77891: 2.5%; Cl 77491: 0.1%; Cl 77499: 0.1% | |
| Abfüllverhältnis Füllgut/Treibgas: 50:50 (Gew%) | |
| Treibgas: Propan/Butan: 2.7 | |

### Beispiel 49

Körperspray (Beine, Arm Gesicht, Fuß und/oder Rücken zur optischen Veränderung des Aussehens der Haut

| Füllgut | Gew.-% |
|---|---|
| PEG-45/Dodecyl Glycol Copolymer | 1,60 |
| Glyceryl Isostearat | 1,60 |
| Octyldodecanol | 10,00 |
| Dihydroxyaceton | 0,30 |
| Parfüm | q.s. |
| Wasser | Ad 100 |
| Farbpigment: | |
| Cl 77492: 0.3%; C177891: 2.5%; Cl 77491: 0.1%; Cl 77499: 0.1% | |
| Abfüllverhältnis Füllgut/Treibgas: 50:50 (Gew%) | |
| Treibgas: Propan/Butan: 2.7 | |

### Beispiel 50

### Haarspray

| Füllgut | Gew.-% |
|---|---|
| PEG-45/Dodecyl Glycol Copolymer | 1,60 |
| Glyceryl lsostearat | 1,60 |
| Octyldodecanol | 10,00 |
| Haarpflege Polymer | 3,00 |
| Parfüm | q.s. |
| Wasser | Ad 100 |
| Abfüllverhältnis Füllgut/Treibgas: 50:50 (Gew%) | |
| Haarpflegepolymer: Polyvinylpyrolidon/Vinylacetat Copolymer | |
| Treibgas: Propan/Butan: 2.7 | |

## Patentansprüche

1. Bei Raumtemperatur als Aerosol versprühbare W/O-Emulsion, enthaltend
A. eine Fettphase, welche
(c) mindestens 90 Gew.-% bei Raumtemperatur flüssige Ölkomponenten und
(d) höchstens 4 Gew.-% an Substanzen, gewählt aus der Gruppe der C3- bis C4-Ester von C12- bis C18-Alkansäuren, C8- bis C12-Alkanole und Silikonöle,
enthält,
B. 20 bis 85 Gew.-% - bezogen auf das Gesamtgewicht des Füllguts (ohne Treibgas) - Wasser,
C. einen oder mehrere W/O-Emulgatoren,
D. einen oder mehrere Stabilisatoren, gewählt aus der Gruppe PEG-45/Dodecylglycolcopolymer, PEG-22/Dodecylglycolcopolymer und/oder Methoxy PEG-22/Dodecyl Glycol Copolymer und
E. ein oder mehrere lipophile Treibgase.

2. W/O-Emulsion nach Anspruch 1, **dadurch gekennzeichnet, dass** sie einen oder mehrere kosmetische Wirkstoffe enthält.

3. W/O-Emulsion nach einem der vorhergehenden Ansprüche, **dadurch gekennzeichnet, dass** der oder die W/O-Emulgatoren gewählt werden aus der Gruppe: PEG-30 Dipolyhydroxystearat, Decaglycerylheptaoleat, Polyglyceryl-3-Diisostearat, PEG-8 Distearat, Diglycerin Dipolyhydroxystearat, Glycerinisostearat, Sorbitanisostearat, Polyglyceryl-3 Methylglucosedistearat, Fettalkohole mit 8 bis 30 Kohlenstoffatomen, Oligo- oder Polyglycerinether, Cetyl Dimethicon Copolyole, Alkyl Methicon Copolyole, Alkyl Dimethicon Ethoxy Glucoside, polyethoxyliertes hydrogeniertes oder nichthydrogeniertes Ricinusöl, ethoxyliertes Cholesterin, ethoxylierte Fettalkohole, ethoxylierte Sorbitanester, ethoxylierte Fettsäuren.

4. W/O-Emulsion nach Anspruch 3, **dadurch gekennzeichnet, dass** die W/O-Emulgatoren gewählt werden aus der Gruppe PEG-30 Dipolyhydroxystearat, Decaglycerylheptaoleat, Polyglyceryl-3-Diisostearat, Diglycerin Dipolyhydroxystearat, Glyceryl Isostearat, Diglycerinisostearat, Sorbitanisostearat, Sorbitanstearat, Cetylalkohol, Stearylalkohol, Cetearylalkohol, Steareth-2, PEG-2 Stearat und/oder Cetyl PEG/PPG-10/1 Dimethicon

5. W/O-Emulsion nach Anspruch 4, **dadurch gekennzeichnet, dass** eine Kombination aus zwei W/O-Emulgatoren gewählt wird.

6. W/O-Emulsion nach einem der vorhergehenden Ansprüche, **dadurch gekennzeichnet, dass** das Gewichts-Verhältnis von W/O-Emulgatoren zu Stabilisatoren von 1:4 bis 4:1, bevorzugt ungefähr 1 : 1 beträgt.

7. W/O-Emulsion nach einem der vorhergehenden Ansprüche, **dadurch gekennzeichnet, dass** ein oder mehrere Verbindungen als Ölkomponenten gewählt werden aus der Gruppe Dicaprylylcarbonat, Dicaprylylether, 2-Ethylhexylcocoat, Myristylmyristat, Octyldodecanol, Polydecen, Squalan, Dimethicon, Cyclomethicon, C-12-15 Alkyl Benzoat, Triisostearin, Capryl-Caprinsäure-Triglycerid, Di-(2-Ethylhexyl)adipat, Ricinusöl, Avocadoöl, Macadamiaöl, Sojaöl und/oder Jojobaöl.

8. W/O-Emulsion nach Anspruch 2, **dadurch gekennzeichnet, dass** sie als Wirkstoff mindestens ein Flavonglycosid, insbesondere α-Glucosylrutin, Vitamin E und/oder dessen Derivate, Q-10 und/oder dessen Derivate, 8-Hexadecen-1,16-dicarbonsäure und/oder alpha-Liponsäure enthält.

9. W/O-Emulsion nach Anspruch 2, **dadurch gekennzeichnet, dass** sie als Wirkstoff mindestens ein Hautbefeuchtungsmittel enthält.

10. W/O-Emulsion nach Anspruch 9, **dadurch gekennzeichnet, dass** als Wirkstoff ein oder mehrere Verbindungen gewählt aus der Gruppe Glycerin, Chitosan, Fucogel, Milchsäure, Propylenglycol, Dipropylenglycol, Butylenglycol, Harnstoff, Natrium und/oder Kaliumsalze Mannitol, Natriumpyrolidoncarbonsäure, Hyaluronsäure und/oder deren Salze und/oder Aminosäuren und/oder deren Salze eingesetzt werden.

11. WIO-Emulsion nach Anspruch 2, **dadurch gekennzeichnet, dass** sie als Wirkstoff einen oder mehrere desodorierende und/oder antitranspirant wirksame Stoffe enthält.

12. W/O-Emulsion nach Anspruch 11, **dadurch gekennzeichnet, dass** als antitranspirant wirksamer Stoff Atuminiumchlorohydrat und/oder als desodorierend wirksamer Stoff Ethanol, 2-Butyloktansäure, Glycerylmonacaprinat, -Caprylat, Diglycerinmonocaprinat, Octoxyglyerin und/oder beliebige Mischungen gewählt werden.

13. W/O-Emulsion nach Anspruch 2, **dadurch gekennzeichnet, dass** sie als Wirkstoff den antiadhäsiven Stoff Chitosan enthält.

14. W/O-Emulsion nach Anspruch 1, **dadurch gekennzeichnet, dass** mindestens eine der Ölkomponenten aus der Gruppe der Silikonöle und der oder die W/O-Emulgatoren aus der Gruppe der nicht silikonhaltigen Emulgatoren gewählt werden.

15. W/O-Emulsion nach Anspruch 1, **dadurch gekennzeichnet, dass** mindestens eine der Ölkomponenten aus der Gruppe der nicht silikonhaltigen Öle und der oder die W/O-Emulgatoren aus der Gruppe der Silikonemulgatoren gewählt werden.

16. W/O-Emulsion nach Anspruch 1, **dadurch gekennzeichnet, dass** weder die Ölkomponenten noch die Emulgatoren silikonhaltig sind.

17. W/O-Emulsion nach Anspruch 2, **dadurch gekennzeichnet, dass** sie als Wirkstoff eine oder mehrere Verbindungen aus der Gruppe der UV-A-, UV-B- und/oder Breitbandfiltersubstanzen enthält.

18. W/O-Emulsion nach Anspruch 2 **dadurch gekennzeichnet, dass** sie als Wirkstoff eine oder mehrere Verbindungen aus der Gruppe der Haarpflegewirkstoffe enthält.

19. W/O-Emulsion nach Anspruch 2 **dadurch gekennzeichnet, dass** sie als Wirkstoff eine oder mehrere Verbindungen aus der Gruppe der Polymere und/oder quaternären Salze für Haarmittelzubereitungen enthält.

20. W/O-Emulsion nach Anspruch 2 **dadurch gekennzeichnet, dass** als Wirkstoff ein Cetyltrimethylammoniumsalz und/oder Potyvinytpyrotidon/Vinytacetat Copolymer gewählt wird.

21. W/O-Emulsion nach einem der vorstehenden Ansprüche, **dadurch gekennzeichnet, dass** sie zusätzlich farbige und/oder nicht farbige Pigmente, Puderrohstoffe, Selbstbräunungsstoffe, hautaufhellende Stoffe und/oder Füllstoffe enthält.

22. W/O-Emulsion nach einem der vorstehenden Ansprüche, **dadurch gekennzeichnet, dass** sie zusätzlich Ethanol enthält.

23. W/O-Emulsion nach einem der vorstehenden Ansprüche, **dadurch gekennzeichnet, dass** als Treibgas einzeln oder in Kombination Propan, Propen, n-Butan, iso-Butan, iso-Buten, n-Pentan, Penten, iso-Pentan, iso-Penten, Methan, Ethan, Dimethylether, Stickstoff, Luft, Kohlendioxid, Sauerstoff, Stickstoffoxide, Lachgas, 1,1,1,3 Tetrafluorethan, Heptafluoro-n-propan, Perfluorethan, Monochlordifluormethan und/oder 1,1-Difluorethan gewählt wird.

24. W/O-Emulsion nach einem der vorstehenden Ansprüche, **dadurch gekennzeichnet, dass** als Treibgas Propan, Butan, iso-Butan oder Mischungen daraus eingesetzt werden.

25. W/O-Emulsion nach einem der vorstehenden Ansprüche, **dadurch gekennzeichnet, dass** das Gewichtsverhältnis von Füllgut zu Treibgas gewählt wird von 20:80 bis 50 : 50.

26. Nicht-therrapeutische Verwendung einer W/O-Emulsion nach einem der vorstehenden Ansprüche als Körperspray zur Veränderung des optischen Aussehens der eingesprühten Körperpartien, insbesondere der Füße, Oberarme, Beine, des Gesichts, Nackens, Haar, Gesäßes und/oder der Rückenpartie.

27. Nicht-therapeutische Verwendung nach Anspruch 26 zur Selbstbräunung.

28. Nicht-therapeutische Verwendung nach Anspruch 26 zur Hautaufhellung.

29. Verwendung der W/O-Emulsion nach einem der Ansprüche 11 oder 12 als Deodorant und/oder Antitranspirant.

30. Verwendung einer W/O-Emulsion nach Anspruch 17 zur Herstellung eines Sonnenschutzmittels.

31. Nicht-therapeutische Verwendung einer W/O-Emulsion nach einem der Ansprüche 1 bis 25 zur Hautpflege.

32. Verwendung einer W/O-Emulsion nach einem der Ansprüche 1 bis 25 zur Haarpflege.

## Claims

1. W/O emulsion sprayable at room temperature as aerosol, comprising
A. a fatty phase which comprises
(a) at least 90% by weight of oil components liquid at room temperature and
(b) at most 4% by weight of substances chosen from the group of C3- to C4-esters of C12- to C18-alkanoic acids, C8- to C12-alkanols and silicone oils,
B. 20 to 85% by weight - based on the total weight of the filling material (without propellant gas) - of water,
C. one or more W/O emulsifiers,
D. one or more stabilizers chosen from the group PEG-45/dodecyl glycol copolymer, PEG-22/dodecyl glycol copolymer and/or methoxy PEG-22/dodecyl glycol copolymer and
E. one or more lipophilic propellant gases.

2. W/O emulsion according to Claim 1, **characterized in that** it comprises one or more cosmetic active ingredients.

3. W/O emulsion according to one of the preceding claims, **characterized in that** the W/O emulsifiers are chosen from the group: PEG-30 dipolyhydroxystearate, decaglyceryl heptaoleate, polyglyceryl-3 diisostearate, PEG-8 distearate, diglycerol dipolyhydroxystearate, diglycerol isostearate, sorbitan isostearate, polyglyceryl-3 methylglucose distearate, fatty alcohols having 8 to 30 carbon atoms, oligo- or polyglycerol ethers, cetyl dimethicone copolyols, alkyl methicone copolyols, alkyl dimethicone ethoxy glucosides, polyethoxylated hydrogenated or nonhydrogenated castor oil, ethoxylated cholesterol, ethoxylated fatty alcohols, ethoxylated sorbitan esters, ethoxylated fatty acids.

4. W/O emulsion according to Claim 3, **characterized in that** the W/O emulsifiers are chosen from the group PEG-30 dipolyhydroxystearate, decaglyceryl heptaoleate, polyglyceryl-3 diisostearate, diglycerol dipolyhydroxystearate, glyceryl isostearate, diglycerol isostearate, sorbitan isostearate, sorbitan stearate, cetyl alcohol, stearyl alcohol, cetearyl alcohol, steareth-2, PEG-2 stearate and/or cetyl PEG/PPG-10/1 dimethicone.

5. W/O emulsion according to Claim 4, **characterized in that** a combination of two W/O emulsifiers is chosen.

6. W/O emulsion according to one of the preceding claims, **characterized in that** the weight ratio of W/0 emulsifiers to stabilizers is from 1:4 to 4:1, preferably approximately 1:1.

7. W/O emulsion according to one of the preceding claims, **characterized in that** one or more compounds as oil components are chosen from the group dicaprylyl carbonate, dicaprylyl ether, 2-ethylhexyl cocoate, myristyl myristate, octyldodecanol, polydecene, squalane, dimethicone, cyclomethicone, C12-15 alkyl benzoate, triisostearin, caprylic/capric triglyceride, di(2-ethylhexyl) adipate, castor oil, avocado oil, macadamia oil, soya oil and/or jojoba oil.

8. W/O emulsion according to Claim 2, **characterized in that** it comprises as active ingredient at least one flavone glycoside, in particular α-glucosylrutin, vitamin E and/or derivatives thereof, Q-10 and/or derivatives thereof, 8-hexadecene-1,16-dicarboxylic acid and/or alpha-lipoic acid.

9. W/O emulsion according to Claim 2, **characterized in that** it comprises as active ingredient at least one skin moisturizer.

10. W/O emulsion according to Claim 9, **characterized in that** the active ingredient used is one or more compounds chosen from the group glycerol, chitosan, Fucogel, lactic acid, propylene glycol, dipropylene glycol, butylene glycol, urea, sodium and/or potassium salts, mannitol, sodium pyrrolidone carboxylic acid, hyaluronic acid and/or salts thereof and/or amino acids and/or salts thereof.

11. W/O emulsion according to Claim 2, **characterized in that** it comprises as active ingredient one or more deodorant and/or antiperspirant substances.

12. W/O emulsion according to Claim 11, **characterized in that** the antiperspirant substance chosen is aluminium chlorohydrate and/or the deodorant substance chosen is ethanol, 2-butyloctanoic acid, glyceryl monocaprate, -caprylate, diglycerol monocaprate, octoxyglycerol and/or any mixtures.

13. W/O emulsion according to Claim 2, **characterized in that** it comprises as active ingredient the anti-adhesive substance chitosan.

14. W/O emulsion according to Claim 1, **characterized in that** at least one of the oil components is chosen from the group of silicone oils and the w/O emulsifier or emulsifiers are chosen from the group of non-silicone-containing emulsifiers.

15. W/O emulsion according to Claim 1, **characterized in that** at least one of the oil components is chosen from the group of non-silicone-containing oils and the W/O emulsifier or emulsifiers are chosen from the group of silicone emulsifiers.

16. W/O emulsion according to Claim 1, **characterized in that** neither the oil component nor the emulsifiers are silicone-containing.

17. W/O emulsion according to Claim 2, **characterized in that** it comprises as active ingredient one or more compounds from the group of UV-A, UV-B and/or broadband filter substances.

18. W/O emulsion according to Claim 2, **characterized in that** it comprises as active ingredient one or more compounds from the group of haircare active ingredients.

19. W/O emulsion according to Claim 2, **characterized in that** it comprises as active ingredient one or more compounds from the group of polymers and/or quaternary salts for hair composition preparations.

20. W/O emulsion according to Claim 2, **characterized in that** the active ingredient chosen is a cetyltrimethylammonium salt and/or polyvinylpyrrolidone/vinyl acetate copolymer.

21. W/O emulsion according to one of the preceding claims, **characterized in that** it additionally comprises coloured and/or noncoloured pigments, powder raw materials, self-tanning substances, skin-lightening substances and/or fillers.

22. W/O emulsion according to one of the preceding claims, **characterized in that** it additionally comprises ethanol.

23. W/O emulsion according to one of the preceding claims, **characterized in that** the propellant gas chosen is, individually or in combination, propane, propene, n-butane, isobutane, isobutene, n-pentane, pentene, isopentane, isopentene, methane, ethane, dimethyl ether, nitrogen, air, carbon dioxide, oxygen, nitrogen oxides, nitrous oxide, 1,1,2,3-tetrafluoroethane, heptafluoro-n-propane, perfluoroethane, monochlorodifluoromethane and/or 1,1-difluoroethane.

24. W/O emulsion according to one of the preceding claims, **characterized in that** the propellant gas used is propane, butane, isobutane or mixtures thereof.

25. W/O emulsion according to one of the preceding claims, **characterized in that** the weight ratio of filling material to propellant gas is chosen to be from 20:80 to 50:50.

26. Nontherapeutic use of a W/O emulsion according to one of the preceding claims as body spray for changing the optical appearance of the sprayed areas of the body, in particular the feet, upper arms, legs, face, neck, hair, bottom and/or part of the back.

27. Nontherapeutic use according to Claim 26 for self-tanning.

28. Nontherapeutic use according to Claim 26 for lightening the skin.

29. Use of the W/O emulsion according to one of Claims 11 or 12 as deodorant and/or antiperspirant.

30. Use of a W/O emulsion according to Claim 17 for producing a sunscreen.

31. Nontherapeutic use of a W/O emulsion according to one of Claims 1 to 25 for skincare.

32. Use of a W/O emulsion according to one of Claims 1 to 25 for haircare.

## Revendications

1. Emulsion E/H pulvérisable à température ambiante sous forme d'un aérosol, contenant
A. une phase grasse, qui contient
(a) au moins 90% en poids de composants huileux liquides à température ambiante et
(b) au maximum 4% en poids de substances choisies dans le groupe des esters en C₃ à C₄ d'acides alcanoïques en C₁₂ à C₁₈, des alcanols en C₈ à C₁₂ et des huiles de silicone,
B. 20 à 85% en poids - par rapport au poids total du produit de remplissage (sans gaz propulseur) - d'eau,
C. un ou plusieurs émulsifiants E/H,
D. un ou plusieurs stabilisateurs, choisis dans le groupe formé par le copolymère de PEG-45/dodécylglycol, le copolymère de PEG-22/dodécylglycol et/ou le copolymère de méthoxy-PEG-22/dodécylglycol et
E. un ou plusieurs gaz propulseurs lipophiles.

2. Emulsion E/H selon la revendication 1, **caractérisée en ce qu'**elle contient une ou plusieurs substances actives cosmétiques.

3. Emulsion E/H selon l'une quelconque des revendications précédentes, **caractérisée en ce que** les émulsifiants E/H sont choisis dans le groupe formé par : le dipolyhydroxystéarate de PEG-30, l'heptaoléate de décaglycéryle, le diisostéarate de polyglycéryle-3, le distéarate de PEG-8, le dipolyhydroxystéarate de diglycérol, l'isostéarate de glycérol, l'isostéarate de sorbitane, le méthylglucosedistéarate de polyglycéryle-3, les alcools gras comprenant 8 à 30 atomes de carbone, les oligoglycéroléthers ou les polyglycéroléthers, les copolyols cétyl-diméthicone, les copolyols alkyl-méthicone, les alkyl-diméthicone-éthoxy-glucosides, l'huile de ricin polyéthoxylée, hydrogénée ou non hydrogénée, le cholestérol éthoxylé, les alcools gras éthoxylés, les esters de sorbitane éthoxylés, les acides gras éthoxylés.

4. Emulsion E/H selon la revendication 3, **caractérisée en ce que** les émulsifiants E/H sont choisis dans le groupe formé par le dipolyhydroxystéarate de PEG-30, l'heptaoléate de décaglycéryle, le diisostéarate de polyglycéryle-3, le dipolyhydroxystéarate de diglycérol, l'isostéarate de glycéryle, l'isostéarate de diglycérol, l'isostéarate de sorbitane, le stéarate de sorbitane, l'alcool cétylique, l'alcool stéarylique, l'alcool cétéarylique, le Steareth-2, le stéarate de PEG-2 et/ou le cétyl-PEG/PPG-10/1 diméthicone.

5. Emulsion E/H selon la revendication 4, **caractérisée en ce qu'**on choisit une combinaison de deux émulsifiants E/H.

6. Emulsion E/H selon l'une quelconque des revendications précédentes, **caractérisée en ce que** le rapport pondéral des émulsifiants E/H aux stabilisateurs est de 1:4 à 4:1, de préférence d'environ 1:1.

7. Emulsion E/H selon l'une quelconque des revendications précédentes, **caractérisée en ce qu'**un ou plusieurs composés, en tant que composants huileux, sont choisis dans le groupe formé par le carbonate de dicaprylyle, le dicaprylyléther, le cocoate de 2-éthylhexyle, le myristate de myristyle, l'octyldodécanol, le polydécène, le squalane, le diméthicone, le cyclométhicone, le benzoate d'alkyle en C₁₂ à C₁₅, la triisostéarine, le triglycéride de l'acide capryl-caprique, l'adipate de di-(2-éthylhexyle), l'huile de ricin, l'huile d'avocat, l'huile de macadamia, l'huile de soja et/ou l'huile de jojoba.

8. Emulsion E/H selon la revendication 2, **caractérisée en ce qu'**elle contient comme substance active au moins un glycoside de flavone, en particulier 1'α-glucosylrutine, la vitamine E et/ou ses dérivés, la Q-10 et/ou ses dérivés, l'acide 8-hexadécène-1,16-dicarboxylique et/ou l'acide alpha-liponique.

9. Emulsion E/H selon la revendication 2, **caractérisée en ce qu'**elle contient comme substance active au moins un agent d'hydratation de la peau.

10. Emulsion E/H selon la revendication 9, **caractérisée en ce qu'**on utilise comme substance active un ou plusieurs composés choisis dans le groupe formé par le glycérol, le chitosane, le fucogel, l'acide lactique, le propylèneglycol, le dipropylèneglycol, le butylèneglycol, l'urée, les sels sodiques ou potassiques du mannitol, le sel sodique de l'acide pyrrolidonecarboxylique, l'acide hyaluronique et/ou ses sels et/ou les acides aminés et/ou leurs sels.

11. Emulsion E/H selon la revendication 2, **caractérisée en ce qu'**elle contient comme substance active une ou plusieurs substances déodorantes et/ou à activité antiperspirante.

12. Emulsion E/H selon la revendication 11, **caractérisée en ce qu'**on choisit comme substance à activité antiperspirante le chlorhydrate d'aluminium et/ou comme substance active déodorante l'éthanol, l'acide 2-butyloctanoïque, le monocaprinate de glycéryle, le monocaprylate de glycéryle, le monocaprinate de diglycérol, l'octoxyglycérol et/ou des mélanges quelconques.

13. Emulsion E/H selon la revendication 2, **caractérisée en ce qu'**elle contient comme substance active la substance anti-adhésive chitosane.

14. Emulsion E/H selon la revendication 1, **caractérisée en ce qu'**on choisit au moins un des composants huileux dans le groupe des huiles de silicone et le ou les émulsifiants dans le groupe des émulsifiants E/H ne contenant pas de silicone.

15. Emulsion E/H selon la revendication 1, **caractérisée en ce qu'**on choisit au moins un des composants huileux dans le groupe des huiles ne contenant pas de silicone et le ou les émulsifiants E/H dans le groupe des émulsifiants à base de silicone.

16. Emulsion E/H selon la revendication 1, **caractérisée en ce que** ni les composants huileux ni les émulsifiants ne contiennent du silicone.

17. Emulsion E/H selon la revendication 2, **caractérisée en ce qu'**elle contient comme substance active un ou plusieurs composés du groupe des substances filtre des UV-A, des UV-B et/ou à large bande.

18. Emulsion E/H selon la revendication 2, **caractérisée en ce qu'**elle contient comme substance active un ou plusieurs composés du groupe des substances actives de soin des cheveux.

19. Emulsion E/H selon la revendication 2, **caractérisée en ce qu'**elle contient comme substance active un ou plusieurs composés du groupe des polymères et/ou des sels quaternaires destinés aux compositions d'agents pour cheveux.

20. Emulsion E/H selon la revendication 2, **caractérisée en ce qu'**on choisit comme substance active un sel de cétyltriméthylammonium et/ou un copolymère de polyvinylpyrrolidone/acétate de vinyle.

21. Emulsion E/H selon l'une quelconque des revendications précédentes, **caractérisée en ce qu'**elle contient en outre des pigments colorés et/ou non colorés, des substances brutes poudreuses, des substances auto-bronzantes, des substances d'éclaircissement de la peau et/ou des charges.

22. Emulsion E/H selon l'une quelconque des revendications précédentes, **caractérisée en ce qu'**elle contient en outre de l'éthanol.

23. Emulsion E/H selon l'une quelconque des revendications précédentes, **caractérisée en ce qu'**elle contient comme gaz propulseurs, seuls ou en combinaison, du propane, du propène, du n-butane, de l'isobutane, de l'isobutène, du n-pentane, du pentène, de l'isopentane, de l'iso-pentène, du méthane, de l'éthane, du diméthyléther, de l'azote, de l'air, du dioxyde de carbone, de l'oxygène, des oxydes d'azote, du gaz hilarant, du 1,1,1,3-tétrafluoroéthane, de l'heptafluoro-n-propane, du perfluoroéthane, du monochlorodifluorométhane et/ou du 1,1-difluoroéthane.

24. Emulsion E/H selon l'une quelconque des revendications précédentes, **caractérisée en ce qu'**on utilise comme gaz propulseur du propane, du butane, de l'isobutane ou des mélanges de ceux-ci.

25. Emulsion E/H selon l'une quelconque des revendications précédentes, **caractérisée en ce que** le rapport pondéral de produit de remplissage à gaz propulseur est choisi de 20 : 80 à 50 : 50.

26. Utilisation non thérapeutique d'une émulsion E/H selon l'une quelconque des revendications précédentes comme spray corporel pour la modification de l'aspect visuel des parties du corps aspergées, en particulier les pieds, les avant-bras, les jambes, le visage, la nuque, les cheveux, les fesses et/ou le dos.

27. Utilisation non thérapeutique selon la revendication 26 pour l'auto-bronzage.

28. Utilisation non thérapeutique selon la revendication 26 pour l'éclaircissement de la peau.

29. Utilisation de l'émulsion E/H selon l'une quelconque des revendications 11 ou 12 comme déodorant et/ou antiperspirant.

30. Utilisation d'une émulsion E/H selon la revendication 17 pour la préparation d'un agent de protection solaire.

31. Utilisation non thérapeutique d'une émulsion E/H selon l'une quelconque des revendications 1 à 25 pour le soin de la peau.

32. Utilisation d'une émulsion E/H selon l'une quelconque des revendications 1 à 25 pour le soin des cheveux.
